(19) Europäisches Patentamt
European Patent Office
Office européen des brevets

(11) EP 2 051 773 B1

(12) # EUROPÄISCHE PATENTSCHRIFT

(45) Veröffentlichungstag und Bekanntmachung des Hinweises auf die Patenterteilung:
**03.11.2010 Patentblatt 2010/44**

(21) Anmeldenummer: **06793083.4**

(22) Anmeldetag: **31.08.2006**

(51) Int Cl.:
***A61N 1/40*** *(2006.01)* ***A61N 2/00*** *(2006.01)*
***A61N 2/02*** *(2006.01)*

(86) Internationale Anmeldenummer:
**PCT/EP2006/065839**

(87) Internationale Veröffentlichungsnummer:
**WO 2008/019710 (21.02.2008 Gazette 2008/08)**

(54) **VORRICHTUNG ZUR STIMULATION MITTELS ELEKTRISCHER UND MAGNETISCHER FELDER SOWIE FELD-APPLIKATOREN HIERFÜR**

DEVICE FOR STIMULATION BY MEANS OF ELECTRIC AND MAGNETIC FIELDS, AND FIELD APPLICATORS FOR THIS PURPOSE

DISPOSITIF DE STIMULATION AU MOYEN DE CHAMPS ÉLECTRIQUES ET DE CHAMPS MAGNÉTIQUES ET APPLICATIONS DE CHAMPS POUR CE DISPOSITIF

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **17.08.2006 PCT/EP2006/065404**

(43) Veröffentlichungstag der Anmeldung:
**29.04.2009 Patentblatt 2009/18**

(73) Patentinhaber: **High Tech Health International Limited**
**Burpengary, QLD 4505 (AU)**

(72) Erfinder:
- **PENNY, Stewart**
  **Maroochydore Bc, Queensland 4558 (AU)**
- **KRAUSS, Manfred**
  **09127 Chemnitz (DE)**
- **FISCHER, Roland**
  **78465 Konstanz (DE)**
- **SCHMIDT, Werner**
  **09117 Chemnitz (DE)**
- **MÖBIUS, Mario**
  **09573 Leubsdorf (DE)**

(74) Vertreter: **Meissner, Bolte & Partner**
**Anwaltssozietät GbR**
**Widenmayerstrasse 48**
**80538 München (DE)**

(56) Entgegenhaltungen:
**DE-A1-102004 024 655**
**US-A- 4 889 526**
**US-A- 5 156 587**
**US-A- 5 899 922**
**US-A1- 2005 080 459**
**US-B1- 6 675 047**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 051 773 B1

## Beschreibung

Die Erfindung betrifft eine Vorrichtung zur Stimulation mittels elektrischer und magnetischer Felder sowie Feld-Applikatoren hierfür.

Wenn biologische Vorgänge durch natürliche elektromagnetische Felder verursacht und beeinflusst werden, wie es u. a. von König [Unsichtbare Umwelt. Der Mensch im Spielfeld elektromagnetischer Kräfte. Eigenverlag Herbert L König, München 1986], Marino [Modern Bioelectricity. Marcel Dekker New York and Basel 1988] und Krauß [QRS-Magnetfeldtherapie - Gegenwart und Zukunft (Plenarvortrag); Tagungsband "QRS-Magnetfeldtherapie - Gegenwart und Zukunft. 1. Internationales Symposium Quantenmedizin in Forschung und Praxis. Darmstadt/Weiterstadt 2. April 2001; "Die natürlichen elektromagnetischen Signale in unserer Umwelt und deren Simulation als QRS®-Magnetfeldtherapie". Vortrag auf dem Norddeutschen Kongress für komplementäre Medizin, 22.-23.6.2002 Wilhelmshaven sowie "Die Mikrostrom-CellVAS®-Therapie" (Mitteilung des Unternehmens Software + Systeme Erfurt GmbH / Germany 2004)] gezeigt wurde, ist es möglich, mit speziellen Feldern die Natur weitestgehend nachzuahmen und bei Vorliegen eines Defizits damit Mensch und Tier zu stimulieren. In diesem Sinne sind auch die von Itil [Quantitative pharmacoelectroencephalography. Use of computerized cerebral biopotentials in psychotropic drug research. In: Itil, T.M.(Ed.): Modern Problems of Pharmacopsychiatry, Vol. 8: Psychotropic Drugs and the Human EEG. Karger, Basel 1974] sowie Fink [Cerebral electrometry - quantitative EEG applied to human psycho-pharmacology. In: Dolce, G. and H. Künkel (Eds): CEAN-computerized EEG-Analysis. Fischer, Stuttgart/New York 1975] aufgestellten und u. a. mittels Pharmako-EEG [Herrmann, W. M. und E. Schärer: Das Pharmako-EEG. Grundlagen, Methodik, Anwendung. Landsberg / Lech / Germany: ecomed 1987 (ISBN 3-609-64170-3)] bewiesenen Hypothesen zu bewerten, nach der

- gleiche EEG-Veränderungen (EEG...Elektroenzephalogramm) zu den gleichen Stimulus-Effekten führen bzw.
- gleiche Stimulus-Effekte mit den gleichen EEG-Veränderungen zusammenhängen.

Figur 1 zeigt die bisher bekannten Hauptfrequenzbereiche physikalischer Stimulationen.

Fournier [Description des installations dúne station dénregistrement des variations tre's rapides du champ magne'tique terrestre; extrait des Comptes Rendus des seánces de l 'Acade'mie des Sciences, t. 251, p.671-673 seánce du Ier aout 1960] berichtet über durchgeführte Erdmagnetfeldmessungen, wo Schwingungen mit einer Variationsbreite der zugehörigen Periodendauern von 30 Sekunden (= 0,03 Hz) bis 0,025 Sekunden (= 40 Hz) ermittelt wurden. Die obere Frequenzgrenze entspricht dabei der des EEG und EKG (EKG...Elektrokardiogramm). König [Unsichtbare Umwelt. Der Mensch im Spielfeld elektromagnetischer Kräfte. Eigenverlag Herbert L König, München 1986] entdeckte, dass mit der Herztätigkeit zusammenhängende Magnetfelder im Frequenzbereich von 0,1 bis 40 Hz liegen. Demnach ist die obere Frequenzgrenze bei Erdmagnetfeld, EKG und EEG gleich. Dies wird auch für die untere Frequenzgrenze gelten, Arbeiten dazu sind nicht bekannt.

Das aus den Erdmagnetfeldmessungen durch Fournier abgeleitete Amplitudenspektrum zeigte eine Resonanzstelle bei der Schumann- bzw. EEG-$\alpha$-Frequenz 10 Hz. Dominant waren jedoch Schwingungen mit der Periodendauer von 4,5 Sekunden, die einer Frequenz von 0,22 Hz entspricht. Dieser Wert stimmt mit dem Bereich der menschlichen Atmung bzw. des Parasympathikus als Komponente des autonomen Nervensystems überein, wie bei Schmidt und Thews [Human Physiologie. Second, Completely Revised Edition, Springer Berlin Heidelberg New York 1989] ersichtlich ist. Hiernach liegt die mittlere Atmungsfrequenz des Erwachsenen unter Ruhebedingungen bei 14 Atemzügen / min (= 0,23 Hz), wobei Variationsbreiten von 9 ... 18 / min [= 0,15 ... 0,3 Hz] auftreten.

Persinger [Possible Cardiac Driving by an External Rotating magnetic Field. Int. J. Biometeor. Vol. 17, No. 3, pp. 263-266, 1973] untersuchte die Möglichkeit der Steuerung der Herztätigkeit bei Ratten durch ein externes mit 0,5 Hz rotierendes Magnetfeld der Stärke von 10-20 Gauß. Dabei ergaben sich signifikant längere RR-Intervalle (RR-Intervall...aus den Gipfeln zweier aufeinanderfolgender R-Zacken im EKG abgeleitetes zeitliches Abstandsmaß, das der Dauer einer Herzperiode und damit der reziproken Herzfrequenz entspricht) als bei den Kontrollgruppen. Das ist offensichtlicher Ausdruck für eine Aktivierung des Parasympathikus, der die Herzfrequenz bekanntermaßen reduziert [Thews, G et R Schmidt: Human Physiologie. Second, Completely Revised Edition, Springer Berlin Heidelberg New York 1989], auch bei der Ratte.

Friedmann et al. [Friedmann, H., R. O. Becker and C. H. Bachmann: Effect of Magnetic Fields on Reaction Time Performance. Nature Vol. 213, No. 5079, pp. 949-950, 1967] untersuchten die Wirksamkeit von extrem langsamen Schwankungen von Magnetfeldern auf die Reaktionszeit des Menschen. Als Ergebnisse stellten sich ein:

- Statische Felder der Stärke von 0,5 mT bzw. 1,7 mT bewirken keinen statistisch gesicherten Einfluß.
- Ein statisches Feld in Kombination mit einem Wechselfeld erbrachte jedoch statistisch gesicherte Ergebnisse: Die mit einem Feld der Parasympathikus-Frequenz 0,2 Hz behandelten Testpersonen hatten gegenüber der Sympathikus-Frequenz 0,1 Hz eine längere Reaktionszeit.

Letztere Ergebnisse zeigen, daß die Frequenz von 0,1 Hz die periphere Mikrozirkulation verändern kann, mit der bekannten Traube-Hering-Welle sowie einer Sympathikusaktivierung übereinstimmt, während 0,2 Hz mit der Atmungsfrequenz bzw. dem Parasympathikus korrelieren.

Wie aus der Physiologie des Menschen bekannt ist [Thews, G et R Schmidt: Human Physiologie. Second, Completely Revised Edition, Springer Berlin Heidelberg New York 1989], sind alle Organe des Körpers, außer der Skelettmuskulatur, von dem vegetativen / autonomen Nervensystem innerviert. Neben dem Einfluß auf die Funktion der inneren Organe auf dem humoralen Weg besteht ein zweiter Weg zur Steuerung der Zellfunktionen: Das vegetative Nervensystem über seine Komponenten Sympathikus und Parasympathikus, die durch Signale der Frequenzbereiche 0,05 ...0,15 Hz bzw. von 0,15 ...0,30 Hz [Thews, G et R Schmidt: Human Physiologie. Second, Completely Revised Edition, Springer Berlin Heidelberg New York 1989] beeinflussbar sind.

Somit wird das vegetative Nervensystem zu einem "Steuerungssystem" für die peripheren Gefäße, Herz, Bronchien, Darm, Niere, Genitalien u. a.

Auf Grund des Wissens über die positiven Auswirkungen von auf den menschlichen Körper einwirkenden elektrischen und magnetischen Feldern sind bereits Vorrichtungen zum Ausführen einer "Eletromagnetischen Feldtherpie" bekannt. Gemäß der Lehre nach US 6,675,047 B1 ist beispielsweise eine Vorrichtung zur Stimulation mittels elektromagnetischer Felder bekannt, wobei die Vorrichtung zum einen Mittel zur Stimulation innerhalb der physiologischen Frequenzbereiche von 0,1 bis 100 Hz umfasst und zum anderen Mittel zum Wobbeln oder Sweepen in Stufen im soeben genannten Frequenzbereich mit 0,01 Hz-Stufen aufweist.

Aus dem Vorgenannten ist es daher Aufgabe der Erfindung, eine weiterentwickelte Vorrichtung zur Stimulation mittels elektrischer und magnetischer Felder sowie hierfür besonders geeignete Feld-Applikatoren anzugeben, wobei mit der Vorrichtung zu gewährleisten ist, dass eine quasi Steuerung des vegetativen Nervensystems erfolgen kann.

Die Lösung der Aufgabe der Erfindung erfolgt gemäß den Merkmalen des Patentanspruchs 1, wobei die Unteransprüche mindestens zweckmäßige Ausgestaltungen und Weiterbildungen darstellen.

Anstelle einer festen Frequenz 0,1 bzw. 0,23 Hz wird der gesamte sympathische [0,05 ...0,15 Hz] oder parasympathische [0,16 ...0,30 Hz] Frequenzbereich in Form einer von außen aufgeprägten Stimulationsfrequenz zeitlich nacheinander in einer beliebig feinen Abstufung durchfahren (gewobbelt, gesweept). Dabei werden zweckmäßigerweise als Stufen dieser Bereiche vorgegeben 0,05; 0,06; 0,07; 0,08; 0,09; 0,1; 0,11; 0,12; 0,13; 0,14, 0,15 Hz bzw. 0.16; 0,17; 0,18; 0,19; 0,20; 0,21; 0,22; 0,23; 0,24; 0,25; 0,26; 0,27; 0,28; 0,29 und 0,30 Hz. Dabei sind als jeweilige Stimulationszeit 30 Sekunden ausreichend. Die Mittelwerte von 0,1 Hz (Sympathikus, Blutdruckperiodik, Traube-Hering-Schwingung) bzw. 0,23 Hz (Atmung, Parasympathikus) werden jedoch mit etwa 4 Minuten länger auf das autonome Nervensystem aufgeprägt.

Erfindungsgemäß erfolgt weiterhin eine kontinuierliche messtechnische Erfassung der Blutdruck- bzw. Atmungsperiodik sowie ein ständiger Vergleich mit den entsprechenden Sollwerten 0,1 bzw. 0,23 Hz. In Anlehnung an regelungstechnische Verfahren wird durch die Stimulation des Nervensystems mit einem Wechselfeld elektrischer oder magnetischer Energie eine Annäherung der gemessenen Ist-Werte an die optimalen Sollwerte angestrebt.

Damit ist erfindungsgemäß realisiert, wie mit eingekoppelten Wechselfeldern, die diese Frequenzen bzw. Frequenzbereiche um 0,1 bzw. 0,23 Hz enthalten, das menschliche autonome Nervensystem stimulierend steuerbar wird.

Im Bereich des Rückenmarks als wesentliche Komponente des Zentralnervensystems befinden sich die entscheidenden Abschnitte zur möglichen Einkopplung der Informationsparameter vor allem für Sympathikus und Parasympathikus mittels physikalischer Stimulation, die erfindungsgemäß über entsprechend konstruktiv zu gestaltende Feld-Applikatoren stimuliert werden.

Dies wird u. a. dadurch erreicht, dass für den Oberkörperbereich das Feld-Applikatorsystem so gestaltet wird, dass sich eine Konzentration des magnetischen Flusses in der oberen Körpermitte des Probanden (Bereich Zentralnervensystem) einstellt. Ebenfalls angepasst an die physiologischen Verhältnisse beim Menschen wird für die untere Körperhälfte das zugehörige Applikatorsystem so realisiert, dass die Verteilung der Feldenergie auf beide Beinbereiche, deren Mitten einen bestimmten Abstand aufweisen, erfolgt und sich somit zwei Feldstärke-Maxima ergeben.

Durch Kombination einer positiven oder negativen E-Feld-Komponente mit einer Steuerungsfrequenz des autonomen Nervensystems sowie übrigen bekannten Stimulationsfrequenzen lassen sich typische Herz-Kreislauf-Stimulationsprogramme erstellen.

Unter der E-Feld-Komponente soll dabei erfindungsgemäß ein zwischen Kopf- und Fußbereich erzeugtes elektrisches Feld verstanden werden. Dieses E-Feld stimuliert im Gegensatz zu dem Magnetfeld Potentialunterschiede. Um den Vorteil der Stimulation über die elektrischen Potentiale mit dem Eintrag der Magnetfeld-Wirkungen zu kombinieren, wird ein Feld-Applikator erfindungsgemäß mit einer Kombination aus einer magnetfelderzeugenden Spulenanordnung und einer das elektrische Feld erzeugenden Elektrodenanordnung ausgestattet. Hierbei kann die das elektrische Feld erzeugende Elektrode gleichzeitig als magnetfelderzeugende Spule gestaltet werden.

Hierin besteht ein grundsätzlicher Unterschied zu herkömmlichen Stimulationsrealisierungen mittels magnetischer Felder, da bei letzteren zusätzlich zum Wechselfeld zwar ein zeitlich unveränderlicher Anteil (DC-Anteil) im Stimulationssignal generiert werden kann, dieser jedoch nur ein gleich bleibendes Magnetfeld und keinen Potentialunterschied zwischen dem kopfnahen Bereich und der Kreislauf-Peripherie erzeugt.

Während die Grundformen bisheriger Stimuli-Signale prinzipiell ähnlich sind und beispielsweise aus einer Mäanderfunktion mit ansteigender e-Funktion bzw. ansteigendem überlagerten Sinus bestehen, dabei z. B. durch entsprechende Aneinanderreihung einer Grundfunktion zu einem Pulspaket auch Frequenzen im EEG-Frequenzbereich realisierbar sind, soll erfindungsgemäß eine Addition von festgelegten Sinusschwingungen mit der entsprechenden niederfrequenten sympathischen oder parasympathischen Steuerfrequenz als Basisschwingung Anwendung finden.

Dieser Steuerfrequenz werden neben der E-Feld-Komponente ausgewählte n Frequenzen des EEG-Bereiches sowie m Frequenzen, die biochemische Reaktionen hervorrufen, überlagert. So wurde erfindungsgemäß mittels durchgeführter Signal-Frequenzoptimierung erkannt, dass eine im Blutdunkelfeld auftretende Aggregation von Erythrozyten vor allem durch Frequenzen im Bereich von ca. 250 bis 1500 Hz auflösbar wird. Als optimale Beziehung wurde erfindungsgemäß hierfür eine Fourier-Reihe mit der Grundschwingung von ca. 250 Hz sowie den Oberschwingungen von ca. 500, 750, 1000, 1250, 1500, ... Hz ermittelt, wenn die Amplituden dieser Oberschwingungen die relativen Anteile ½, $^1/_3$, ¼, 1/5, 1/6... betragen und eine Gesamt-Signal-Phasenverschiebung von 180° realisiert wird. Bezeichnet man diese Stromkomponente, die über das entsprechende Magnetfeld die Normalisierung der Erythrozytenaggregation hervorrufen soll, mit

$i_{IIS}$ (t) [iis ...ion injection signal], so erhält man im Falle einer Grundschwingung von 250 Hz als Beispiel (A ... Amplitude der Grundschwingung 250 Hz):

$$i_{IIS}(t) = -A[\sin 2\pi 250t + 0{,}5\sin 2\pi 500t + 0{,}33\sin 2\pi 750t + 0{,}25\sin 2\pi 1000t + \\ + 0{,}2\sin 2\pi 1250t + 0{,}17\sin 2\pi 1500t + ...]$$

Das so erzeugte Signal wird den bekannten Frequenzen des EEG-Bereiches sowie der entsprechenden Steuerfrequenz des autonomen Nervensystems überlagert. Da die Erythrozyten, insbesondere deren Membran, ein Schwingungssystem darstellen und die optimale Frequenz (Resonanzfrequenz) bei etwa 1 kHz liegt, kann sich durch Resonanzwirkung an der Membran eine derartige Erythrozytenvereinzelung einstellen. Im Falle einer (pathologischen) Erythrozytenaggregation verschlechtern sich die Fließeigenschaften des Blutes. Des weiteren reduziert sich durch eine auftretende Dämpfung die Resonanzfrequenz dieses Systems, wie aus der Theorie der (linearen) Systeme bekannt ist. Erfindungsgemäß wird der möglichen Frequenz-Verstimmung der Blutzellen dadurch begegnet, dass die im Signal enthaltenen Frequenzanteile in einer definierten Bandbreite durchlaufen werden, also in geringem Maße um eine Mittenfrequenz herum pendeln.

Die für die Stimulation vorgesehenen Felder werden über eine spezielle Vorrichtung zur Erzeugung der Felder generiert. Diese Vorrichtung wandelt die in der Signalerzeugungsvorrichtung erzeugten elektrischen Signale in die für die Stimulation vorgesehenen Energieformen um. Dabei sind verschiedene Vorrichtungen in Form einfacher elektrischer Spulen, integriert in einen Matten-Applikator, bekannt. Hier werden verschiedene elektrische Spulen so angeordnet, dass diese ein vorwiegend magnetfeldinduziertes Feld zur Anwendung bringen. Da der Betrieb der Vorrichtung bei Frequenzen unterhalb von 1MHz erfolgt, werden durch die bekannte Vorrichtung nahezu keine elektrischen Felder generiert. Das aus der Elektromagnetik bekannte Gleichgewicht von elektrischem und magnetischem Feld stellt sich erst bei einer Entfernung von weit über der Wellenlänge ein, die selbst bei einer Frequenz von 1MHz noch ca. 300m beträgt. Im unmittelbaren Anwendungsbereich der Vorrichtung erzeugen die bekannten Vorrichtungen demnach nahezu keine elektrische Feldkomponente.

Wie oben dargelegt, ist die Stimulierung besonders wirkungsvoll in der Kombination von elektrischem und magnetischem Feld. Erfindungsgemäß wird deshalb die Vorrichtung zur Felderzeugung aus verschiedenen einzelnen Spulen oder Spulen in Kombination mit elektrisch leitfähigen Gittern oder Flächen aufgebaut, die sowohl die Einspeisung elektrischer Ströme als auch elektrischer Spannungen erlauben.

Insbesondere ist es durch das Zusammenwirken der Elemente möglich, die Kombination der Felder zur Wirkung zu bringen.

Die Erfindung soll anhand von Ausführungsbeispielen und von Figuren näher erläutert werden. Hierbei zeigen:

Figur 1 charakteristische Stimulationsfrequenzen bei elektrischen und magnetischen Feldern [aus Krauß, M: "Die natürlichen elektromagnetischen Signale in unserer Umwelt und deren Simulation als QRS®-Magnetfeldtherapie". Vortrag auf dem Norddeutschen Kongress für komplementäre Medizin, 22.-23.6.2002 Wilhelmshaven],

Figur 2 eine einfache Vorrichtung zur Erzeugung eines elektrischen Feldes kombiniert mit einem magnetischen Feld durch die Umwandlung elektrischer Signale, wobei das elektrische Feld dem magnetischen Feld folgt,

Figur 3 eine erweiterte Beschaltung der Vorrichtung zur Erzeugung eines elektrischen Feldes kombiniert mit einem magnetischen Feld, wobei das elektrische Feld unabhängig vom magnetischen Feld gesteuert werden kann,

Figur 4 eine erweiterte Vorrichtung zur Erzeugung eines elektrischen Feldes kombiniert mit einem magnetischen Feld,

Figur 5 eine spezielle Ausführung der Vorrichtung zur Erzeugung eines elektrischen Feldes kombiniert mit einem magnetischen Feld,

Figur 6 die Verteilung des elektrischen Feldes zwischen der oberen und der unteren Spule bei einer Anordnung nach Figur 3,

Figur 7 die Verteilung des elektrischen Feldes zwischen einer Spannungselektrode und einem Spulensystem bei einer Anordnung nach Figur 4 oder 5,

Figur 8 den Verlauf der magnetischen Flussdichte über einer Spulenanordnung angepasst an die physiologischen Verhältnisse,

Figur 9 eine Ausführung der Vorrichtung zur Erzeugung eines elektrischen Feldes kombiniert mit einem magnetischen Feld in Anlehnung an die Ausführung nach Figur 5,

Figur 10 eine einfache Vorrichtung zur Überprüfung der Funktionsweise der Vorrichtung zur Probandenstimulation mittels spezieller elektrischer und magnetischer Felder sowie optimierter Feld-Applikatoren,

Figur 11 den charakteristischen Verlauf von peripheren Durchblutungsparametern, abgeleitet mit der nichtinvasiven NIRP-Methode vom Messort Fingerbeere bei einem 40jährigen Normalprobanden, bei Veränderung der E-Feld-Komponente eines Stimulationsprogrammes "Vitality" als Ausführungsbeispiel,

Figur 12 eine Schwingungsperiode der niederfrequenten sympathischen (a) sowie parasympathischen (b) Steuerfrequenz als Basisschwingung eines entsprechenden Stimulationsprogramms,

Figur 13 den periodischen Verlauf der definierten höherfrequenteren IIS-Funktion $i_{IIS}$ (t) [iis ... ion injection signal] mit der Periode von 4 ms ≡ 250 Hz und den Frequenzen 250, 500, 750, 1000 und 1250 Hz, wenn die jeweiligen Amplituden dieser Schwingungen die relativen Anteile 1, ½, $^1/_3$, ¼, 1/5, 1/6 aufweisen,

Figur 14 als zu verallgemeinerndes Ausführungsbeispiel ein Ergebnis durchgeführter Blutdunkelfeld-Aufnahmen bei einem 55jährigen Diabetespatienten vor, nach 2, 4 und 12 Minuten Stimulation mit der IIS-Funktion $i_{IIS}$ (t) nach Figur 13,

Figur 15 charakteristische Schwingungsfrequenzen, die der parasympathischen Steuerfrequenz von 0,25 Hz überlagert werden, für ein Stimulationsprogramm "Relax" als Ausführungsbeispiel:

a) Basisschwingung 0,25 Hz mit einer relativen Amplitude von 50 % und einer Relax-Bezugsfrequenz (EEG-Delta-Frequenz) von 2 Hz bei 100 % Amplitudenanteil,
b) EEG-Alpha-Frequenz von 10 Hz mit 50 % und 100 Hz mit 40 % zum Referenzsignal (Bezugsfrequenz) von 2 Hz,
c) Gesamtsignal aus Addition von a) und b) sowie von $i_{IIS}$ (t) [s. Figur 13] für die Stimulationszeit von 4 s, die der Periode der Basisschwingung von 0,25 Hz ≡ 4 s entspricht,

Figur 16 charakteristische Schwingungsfrequenzen, die der parasympathischen Steuerfrequenz von 0,25 Hz überlagert werden, für ein Stimulationsprogramm "Vitality" als Ausführungsbeispiel:

a) Vitality-Bezugsfrequenz von 10 Hz (EEG-Alpha-Frequenz) bei 100 % Amplitudenanteil sowie 100 Hz mit 40 % Amplitudenanteil,
b) Gesamtsignal aus Addition von a) sowie von 0,25 Hz (50 % Amplitudenanteil, s. Figur 12) und von $i_{IIS}$ (t) [s. Figur 13] für die Stimulationszeit von 4 s, die der Periode der Basisschwingung von 0,25 Hz ≡ 4 s entspricht,

und

Figur 17 charakteristische Schwingungsfrequenzen, die der sympathischen Steuerfrequenz von 0,1 Hz überlagert werden, für ein Stimulationsprogramm "Performance" als Ausführungsbeispiel:

a) Performance-Bezugsfrequenz von 10 Hz (EEG-Alpha-Frequenz) bei 100 % Amplitudenanteil und 20 Hz mit 70 % Amplitudenanteil sowie 100 Hz mit 40 % Amplitudenanteil,
b) Gesamtsignal aus Addition von a) sowie von 0,1 Hz (50 % Amplitudenanteil, s. Figur 12) und von $i_{IIS}$ (t) [s. Figur 13] für die Stimulationszeit von 10 s, die der Periode der Basisschwingung von 0,1 Hz ≡ 10 s entspricht.

Die Vorrichtung nach Figur 2 ist dadurch gekennzeichnet, das der Applikator aus zwei Spulensystemen 20 und 30 aufgebaut wird, die räumlich so angeordnet sind, dass einerseits eine Konzentration der Magnetfelder auf verschiedene Bereiche der Einwirkung erreicht wird, andererseits aber auch ein Potentialunterschied zwischen den Spulen zum Aufbau eines spezifischen elektrischen Feldes führt.

Der Potentialunterschied wird durch den Spannungsabfall über einen Widerstand 40, der zwischen Anschluss 22 und Anschluss 31 geschaltet ist, erzeugt. Durch diese Beschaltung wird erreicht, dass das elektrische Feld dem magnetischen Feld folgt. Wird dem Strom zur Erzeugung des Magnetfeldes ein Gleichstrom (DC-Komponente) überlagert, wird zusätzlich die Ausbildung eines konstanten E-Feldes erreicht.

Dargestellt sind in Figur 2 zwei Spulensysteme 20 und 30 mit den Anschlüssen 21 und 22 für das Spulensystem 20 sowie den Anschlüssen 31 und 32 für das Spulensystem 30. Durch die Stromquelle 50 wird ein Strom 51 in die Spulensysteme derart eingespeist, das der Strom zuerst die Spule 20 durchfließt, anschließend über einen Widerstand 40, der zwischen die Anschlüsse 22 und 31 geschaltet ist, in das Spulensystem 30 geleitet wird und anschließend über den Anschluss 32 wieder zur Stromquelle zurück fließt. Die Wirkung des elektrischen Feldes entsteht durch den Spannungsabfall über dem Widerstand, so dass zwischen dem Spulensystem 20 und 30 ein elektrischer Potentialunterschied aufgebaut wird. Der Stromfluss in den Spulensystemen wiederum erzeugt die Wirkung des magnetischen Feldes. Der Strom 51 wird durch die Stromquelle 50 in der Intensität mit den oben beschriebenen Frequenzen moduliert, so dass die elektrischen und magnetischen Felder in beschriebener Weise ihre Wirkung entfalten können.

Die Anordnung nach Figur 3 zeigt eine Erweiterung der Funktionalität des Beispieles nach Figur 2 durch eine veränderte Beschaltung.

Diese erlaubt es, sowohl die elektrische Feldkomponente wie die magnetische Feldkomponenten separat zu steuern. Neben der Verbesserung der Anwendung der Vorrichtung erreicht man zusätzlich eine bessere Energieausnutzung, da die Verluste durch den ohmschen Widerstand entfallen. Außerdem kann der Gleichanteil im Steuersignal für die magnetische Feldkomponente entfallen, da dieser DC-Anteil energiesparend für das elektrische Feld eingesetzt werden kann.

Die Figur 3 zeigt im einzelnen: Zwei Spulensysteme 20 und 30 mit den Anschlüssen 21 und 22 für das System 20 und 31 und 32 für das System 30. Durch die Stromquellen 50 wird ein entsprechend der Zielstellung modulierter Strom in das eine Spulensystem, hier gezeigt das System 30, eingespeist. Die Stromquelle 51 speist des weiteren einen Strom in das System 20 ein und erzeugt in dem Wirkungsbereich dieses Spulensystem ein zweites magnetisches Feld, welches durch die separate Stromquelle auch unabhängig in Intensität oder Frequenzzusammensetzung von dem des ersten Spulensystems gesteuert werden kann. Zwischen beiden Spulensystemen wird mit einer Spannungsquelle 60 eine elektrische Potentialdifferenz erzeugt, so dass sich ein elektrisches Feld zwischen den beiden Spulensystemen ausbilden kann.

In Figur 4 ist eine erweiterte Vorrichtung zur Erzeugung eines elektrischen Feldes kombiniert mit einem magnetischen Feld ersichtlich, wobei für das elektrische Feld und das magnetische Feld eigenständige Elektrodensysteme vorliegen und das magnetische Feld mit einer einzigen Stromquelle und das elektrische Feld mit einer einzigen Spannungsquelle erzeugt werden.

Bei dieser Matten-Applikator-Anordnung ergeben sich zwei Vorteile. Zum einen ist man in der Gestaltung des das Magnetfeld erzeugenden Spulensystems weitgehend frei und kann sich auf die optimale Anwendungszielstellung orientieren. Das elektrische Feld kann in gleicher Weise optimiert werden, so dass beide Felder unabhängig von einander ausgeprägt werden können. Ein weiterer Vorteil gegenüber den Anordnungen nach den Figuren 2 und 3 ist die Konzentration des elektrischen Feldes auf die eine Seite des Applikators, von der die Anwendung ausgeht. Die Rückseite desselben schirmt das elektrische Feld ab, so dass einerseits keine Störung durch beispielsweise elektrische Leitungen unterhalb des Matten-Applikators auftreten können. Auch wird das anwendungsgerecht ausgebildete Feld nicht durch andere metallische Flächen (Metallboden, Stahlarmierungen) gestört.

Speziell zeigt Figur 4 ein Spulensystem 20 mit den Anschlüssen 21 und 22, welches auf einfache Weise in einer Ebene aufgebaut werden kann, die beispielsweise in einer Matte eingebettet ist. In einer zweiten Ebene, die elektrisch isoliert über der Ebene des Spulensystems 20 angeordnet ist, wird eine Elektrode 30 angeordnet, die geschlossen flächig oder durch einzelne leitfähige Bahnen eine Fläche abdeckend ausgeführt sein kann. Die das Magnetfeld steuernde Stromquelle 51 speist den steuernden Strom in die Anschlüsse 21 und 22 ein. Die das elektrische Feld steuernde Spannungsquelle 60 erzeugt ein gesteuertes Potential zwischen der Elektrode 30 und dem Spulensystem 20.

Figur 5 zeigt eine spezielle Ausführung der Vorrichtung zur Erzeugung eines elektrischen Feldes kombiniert mit einem magnetischen Feld, wobei das Spulensystem so ausgeführt ist, dass eine an die Physiologie des Lebewesens, das sowohl ein Mensch als auch ein Tier sein kann, angepasste Feldverteilung entsteht.

In dieser Ausführung wird die Gestaltungsfreiheit der das magnetische Feld erzeugenden Spulen bei einer Ausführung des Applikators nach Figur 4 dargestellt. Figur 5 zeigt das Spulensystem zwischen dem Anschluss 21 und 22 bestehend aus den Teilspulen 201 und 202 sowie 203. Des weiteren ist eine Elektrode 30 für das elektrische Feld dargestellt.

Die Anordnung der Spulen 201 und 202 ist so getroffen, dass der Strom diese in entgegengesetzter Richtung durchfließt. Dadurch wird das Magnetfeld in der Mitte konzentriert, wie aus dem Diagramm der Feldstärkeverteilung in der Figur 8 Schnitt A-A ersichtlich ist. Die Anordnung der Spule 203 ist so getroffen, dass die über den seitlichen Spulenleitern starke Magnetfeldausbildung optimal unter den Extremitäten zur Wirkung kommt, wie das Diagramm der Feldstärkeverteilung in Figur 8 Schnitt B-B zeigt. Die Wirkung der Spannungselektrode ist in Figur 7 als Feldlinien-Diagramm im Schnitt C-C dargestellt.

Die Verteilung des elektrischen Feldes zwischen der oberen und der unteren Spule bei einer Anordnung nach Figur 3 geht aus Figur 6 hervor. Gezeigt sind die Feldlinien, die sich zwischen einem beispielsweise positiven Potential der Spule 20 und einem dementsprechend negativen Potential der Spule 30 ausbilden. Zu erkennen ist die symmetrische Ausbildung nach oben und nach unten.

Die Figur 7 zeigt die Feldlinien eines elektrischen Feldes, welches sich zwischen einer beispielsweise positiven Elektrode und dem dementsprechend negativen Spulensystem einstellt. Die Linien verbinden jeweils die positive, helle Seite mit der negativen, dunklen Seite des Potentials.

Figur 8 zeigt den Verlauf der magnetischen Flussdichte über einer Spulenanordnung angepasst an die gegebenen physiologischen Verhältnisse, wobei das Diagramm der durchgezogenen Linie den Verlauf im Bereich des Schnittes B-B (s. Figur 5) und die unterbrochene Linie den Verlauf im Bereich des Schnittes A-A (s. Figur 5) darstellt. Erkennbar ist die Konzentration der Feldstärke im Schnitt A-A auf den mittleren Bereich, der vorzugsweise im Bereich der Wirbelsäule angeordnet wird, und die Verteilung der Feldstärkemaxima auf zwei Seiten, so dass diese Anordnung für die Extremitäten bevorzugt wird.

Figur 9 zeigt eine Ausführung der Vorrichtung zur Erzeugung eines elektrischen Feldes kombiniert mit einem magnetischen Feld in Anlehnung an die Ausführung nach Figur 5, wobei die Spulen 201, 202 und 203 nur noch aus einer ebenen, flachen Stromschleife bestehen. Diese ebenen, parallelen Strompfade können beispielsweise in einen Matten-Applikator integriert werden, indem durch elektrisch leitfähige Garne in Schuss und/oder Kette die Leitbahnen eingearbeitet werden. Hierbei sind in einem Teil die Leitbahnen derart verbunden, dass ein Spulensystem angepasst an die gegebenen physiologischen Verhältnisse entsteht, wobei jede der Spulen des Systems aus nur einer einzigen Stromschleife besteht.

Der Strom zur Erzeugung des Magnetfeldes wird durch die Stromquelle I in den Anschluss b eingeleitet. Nachdem der Strom durch die Spulen 202 und 201 fließt, wird er in die Spule 203 in den unteren Bereich weitergeleitet und dann über den Anschluß c wieder zur Stromquelle geführt. Mit der Spannungsquelle U wird zwischen der Elektrode 30 und dem Spulensystem 201, 202, 203 ein elektrisches Feld erzeugt, indem die Spannung über den Anschluss a an die Elektrode 30 und über den Anschluß c an das Spulensystem angeschlossen wird.

Um eine einfache Ausführung des Anschlusskabels zu erreichen und eine möglichst geringe Störbeeinflussung der Umgebung zu sichern, wird das Anschlusskabel mit den Leitern a, b und c so ausgeführt, dass die stromführenden Leitungen a und b verdrillt werden und die Leitung c als Abschirmung über die Leitungen a und b gezogen wird. Die Anordnung der Spulen erfolgt vorzugsweise in der Art, dass die Spulen 201 und 202 ihre Wirkung im Bereich der Wirbelsäule eines Menschen oder gegebenenfalls eines Wirbeltieres erlangt und die Spule 203 ihre Wirkung im Bereich der Extremitäten, insbesondere der Beine, erreicht. Um hierfür eine optimale Anordnung zu schaffen, kann der Abstand zwischen den oberen und den unteren Spulen variabel gestaltet werden.

Der Abstand der Längsstrompfade in der Spule 203 ist dem Abstand der Beine anzupassen, so dass die in Figur 8 Schnitt B-B erkennbaren Maxima der magnetischen Flussdichte optimal die Nervenbahnen sowie die peripheren Blutgefäße in den Gliedmaßen stimulieren können.

Figur 10 zeigt eine einfache Vorrichtung zur Überprüfung der Funktionsweise der Vorrichtung zur Erzeugung eines kombinierten elektrischen und magnetischen Feldes mit den für die angestrebte Wirkung zugrunde gelegten Frequenzen.

Diese Vorrichtung beinhaltet eine Spule 100, die mit dem Kondensator 200 einen Resonanzkreis bildet, der auf eine charakteristische Frequenz des Signalgemisches des $i_{IIS}$ - Signals, z. B. 1000 Hz, abgestimmt ist und somit 50 Hz -Stör- und sonstige Signale nicht erfasst. Der Detektor 300, im einfachsten Fall eine Diode, erzeugt aus der in dem Schwingkreis durch das Stimuli-Signal induzierten Wechselspannung ein Steuersignal, welches ggf. noch durch den Verstärker 400 verstärkt wird. Das Steuersignal wird mittels eines Indikators 500 angezeigt, der im einfachsten Fall eine Leuchtdiode sein kann, der aber auch mehrere Anzeigezustände haben kann.

Nach gleichem Algorithmus entsprechend Figur 10 wird eine verallgemeinerte Vorrichtung erfindungsgemäß dergestalt realisiert, dass neben der Vorrichtung zur Überprüfung der Funktionsweise der Vorrichtung zur Erzeugung eines kombinierten elektrischen und magnetischen Feldes mit den für die erstrebte Wirkung zugrunde gelegten Frequenzen gleichfalls und parallel über ein gesondertes System Spule / Resonanzkreis die Erfassung von 50 bzw. 60 Hz - Störsignalen möglich ist.

Aus Figur 11 geht hervor, dass eine Umkehr der Polarität in Verallgemeinerung durchgeführter Messungen zu einer Reduzierung der arteriellen Durchblutungsgüte sowie zu einer Verschlechterung der Blutgefäßelastizität (=Erhöhung des Faser-Stretchings) bei diesem Anwendungsprogramm führt. Dieses Ausführungsbeispiel bestätigt die Abhängigkeit der peripheren Kreislaufparameter von einem E-Feld zwischen kopfnahem und peripherem Bereich.

In Figur 12 ist eine Schwingungsperiode der niederfrequenten sympathischen (a) sowie parasympathischen (b) Steuerfrequenz als Basisschwingung eines entsprechenden Stimulationsprogramms dargestellt. Der sympathischen (0,1 Hz ≡ 10 s Schwingungsperiode) bzw. parasympathischen (0,25 Hz ≡ 4 s Schwingungsperiode) Basisschwingung werden entsprechende höherfrequentere Schwingungen überlagert, wie aus den Figuren 13 sowie 15 bis 17 hervorgeht.

Die IIS-Funktion $i_{IIS}$ (t) nach Figur 13 wird den Stimulationsprogrammen, speziell den Steuerungsfrequenzen 0,1 und 0,25 Hz sowie entsprechenden EEG-Frequenzen und der Frequenz 100 Hz überlagert, um vor allem auftretende (pathologische) Erythrozytenaggregationen aufzulösen und verbesserte Blutfließeigenschaften zu erzielen. Diese Ergebnisse lassen sich aus Kapillarflussmessungen und mikroskopischen Blutdunkelfeld-Aufnahmen ableiten.

Aus Figur 14 ist ersichtlich, wie sich die Erythrozyten unter einer Stimulation mit der IIS-Funktion $i_{IIS}$ (t) nach Figur 13 vereinzeln, nachdem vor der Stimulation offensichtliche pathologische Erythrozytenaggregationen vorlagen. Es wurde erfindungsgemäß erkannt, bei einer Stimulation zunächst nur ein solches IIS-Signal für etwa 4 Minuten wirken zu lassen, anschließend das Stimulations-Anwendungsprogramm zu aktivieren (s. Figuren 15 bis 17).

Während in Figur 15 für das Stimulationsprogramm "Relax" die Frequenz von 2 Hz eine typische EEG-Delta-Frequenz darstellt und sich bei derartigen Delta-Frequenzen hochsignifikante Veränderungen bei Schlaflosigkeit einstellten, liegen zu den 10Hz-Feldern (s. auch die Stimulationsprogramme "Vitality" und "Performance" nach Figur 16 und 17) ebenfalls hochsignifikante Ergebnisse u. a. bei immunbiologischen Reaktionen, Ödemhemmung, Wetterfühligkeit, Verbesserung der Konzentrationsfähigkeit, allgemeine Leistungssteigerung vor.

## Patentansprüche

**1.** Vorrichtung zur Stimulation mittels elektrischer und magnetischer Felder, umfassend

- Mittel zur äußeren physikalischen Stimulation des vegetativen / autonomen Nervensystems als Steuerungskomponente des Herz-Kreiskreislauf-Systems mit den Teilen Sympathikus und Parasympathikus innerhalb der zugehörigen physiologischen Frequenzbereiche 0,05 ... 0,1 ... 0,15 Hz oder 0,15 ... 0,23 ... 0,30 Hz und
- Mittel zur Wobbelung oder Sweepen in Stufen im oben genannten Frequenzbereich mit Stufung 0,05; 0,06; 0,07; 0,08; 0,09; 0,1; 0,11; 0,12; 0,13; 0,14, 0,15 Hz oder 0,16; 0,17; 0,18; 0,19; 0,20; 0,21; 0,22; 0,23; 0,24; 0,25; 0,26; 0,27; 0,28; 0,29 und 0,30 Hz,

**dadurch gekennzeichnet, dass**
die Vorrichtung des Weiteren Mittel zur Addition der charakteristischen Sinusschwingungen und Erzeugen eines positiven oder negativen E-Feldes zwischen Kopf- und Peripherie-Bereich des Probanden zur Bildung eines modularen Gesamt-Stimulationssignals mit der niederfrequenten sympathischen oder parasympathischen Steuerfrequenz als Basisschwingung sowie anwendungstypischen EEG-Frequenzen und höherfrequenteren Sinusschwingungen einer Fourier-Reihe einer Grundschwingung von 250 Hz sowie den Oberschwingungen von 500, 750, 1000, 1250, 1500 Hz aufweist.

**2.** Vorrichtung nach Anspruch 1,
**dadurch gekennzeichnet, dass**
zur Einkopplung der Energie Feld-Applikatoren eingesetzt werden, welche gewährleisten, dass sich im Oberkörperbereich des Probanden eine Konzentration des magnetischen Flusses, der von einem Matten-Applikator erzeugt wird, in der Körpermitte einstellt und unter Zugrundelegung der physiologischen Verhältnisse in der unteren Körperhälfte ein zugehöriger Matten-Applikator so realisiert ist, dass die Verteilung der Feldenergie auf beide Beinbereiche, deren Mitten einen bestimmten Abstand aufweisen, erfolgt und sich somit zwei Feldstärke-Maxima ergeben.

**3.** Vorrichtung nach Anspruch 2,
**dadurch gekennzeichnet, dass**
die Feld-Applikatoren eine Kombination aus einer magnetfelderzeugenden Spulenanordnung und einer das elektrische Feld erzeugenden Elektrodenanordnung umfassen, um den Vorteil der Stimulation über die elektrischen Potentiale mit dem Eintrag der Magnetfeld-Wirkungen zu kombinieren und dabei die das elektrische Feld erzeugende Elektrode gleichzeitig als magnetfelderzeugende Spule gestaltbar ist.

**4.** Vorrichtung nach Anspruch 3,
**dadurch gekennzeichnet, dass**
die Applikationen aus verschiedenen einzelnen Spulen oder Spulen in Kombination mit elektrisch leitfähigen Gittern oder Flächen besteht, die sowohl die Einspeisung elektrischer Ströme als auch elektrischer Spannungen erlauben, wobei durch das Zusammenwirken der Elemente elektrische und magnetische Felder kombiniert zur Wirkung gebracht werden.

**5.** Vorrichtung nach einem der vorangegangenen Ansprüche,
**dadurch gekennzeichnet, dass**
mittels eines auf die Resonanzfrequenz abgestimmten Spule-Kondensator-Schwingkreises, die mit einer charakteristischen Frequenz des Stimulationssignals übereinstimmt und über einen Detektor Anzeigezustände realisiert sind, um die Wirkung der Stimulationsvorrichtung zu überprüfen.

## Claims

**1.** A device for stimulation via electric and magnetic fields, comprising:

- means for external physical stimulation of the vegetative / autonomic nervous system as a control component of the cardiovascular system with the parts of a sympathetic and parasympathetic nervous system within the corresponding physiological frequency ranges 0.05 ... 0.1 ... 0.15 Hz or 0.15 ... 0.23 **...** 0.30 Hz, and
- means for wobbling or sweeping in steps in the aforementioned frequency range with steps 0.05; 0.06; 0.07; 0.08; 0.09; 0.1; 0.11; 0.12; 0.13; 0.14; 0.15 Hz or 0.16; 0.17; 0.18.; 0.19; 0.20; 0.21; 0.22; 0.23; 0.24; 0.25; 0.26; 0.27; 0.28; 0.29 and 0.30 Hz,

**characterized in that**
the device further comprises means for adding the characteristic sinusoidal oscillations and creating a positive or negative E-field between the head and peripheral area of the subject for forming a modular overall stimulation signal with the low-frequency sympathetic or parasympathetic control frequency as a basic oscillation as well as application-typical EEG-frequencies and higher frequency sinusoidal oscillations of a Fourier series of a fundamental oscillation of 250 Hz as well as the harmonic oscillations of 500, 750, 1000, 1250, 1500 Hz.

**2.** Device according to claim 1,
**characterized in that**
field applicators are used for coupling in energy, which ensure that a concentration of the magnetic flux created by a mat applicator appears in the center of the body in an upper body area of the subject and, based on the physiological conditions, a corresponding mat applicator is realized in the lower half of the body such that the distribution of the field energy occurs to both leg areas, with centers thereof being a certain distance in reference to each other, and thus two maximums for field strength result.

**3.** Device according to claim 2,
**characterized in that**
the field applicators comprise a combination of a coil arrangement creating a magnetic field and an electrode arrangement creating the electric field in order to combine the advantage of the stimulation via the electric potentials with the input of the effects of magnetic fields and that thus the electrode creating the electric field can simultaneously be designed as a coil creating the magnetic field.

**4.** Device according to claim 3,
**characterized in that**
the applications comprise various individual coils or coils in combination with electrically conductive grids or surfaces allowing both an introduction of electric currents as well as electric voltages, with the electric and the magnetic fields being allowed to become effective in combination by the cooperation of the elements.

**5.** Device according to one of the preceding claims,
**characterized in that**

by means of a coil capacitor oscillating circuit tuned to the resonance frequency which coincides with a characteristic frequency of the stimulation signal, and via a detector display statuses are realized in order to check the effectiveness of the stimulation device.

## Revendications

1. Dispositif pour la stimulation au moyen de champs électriques et magnétiques, comprenant

    - des moyens pour une stimulation physique extérieure du système nerveux végétatif/autonome à titre de composante de commande du système coeur-circulation avec les parties formées par le sympathique et le parasympathique à l'intérieur des plages de fréquences physiologiques associées 0,05 ... 0,1 ... 0,15 Hz ou 0,15 ... 0,23 ... 0,30 Hz, et
    - des moyens pour appliquer une wobulation ou un glissement par étages dans la plage de fréquences citée ci-dessus avec des pas de 0,05 ; 0,06 ; 0,07 ; 0,08 ; 0,09 ; 0,1 ; 0,11 ; 0,12 ; 0,13 ; 0,14 ; 0,15 Hz ou 0,16 ; 0,17 ; 0,18 ; 0,19 ; 0,20 ; 0,21 ; 0,22 ; 0,23 ; 0,24 ; 0,25 ; 0,26 ; 0,27 ; 0,28 ; 0,29 et 0,30 Hz,

    **caractérisé en ce que**
    le dispositif comprend en outre des moyens pour l'addition d'oscillations sinusoïdales caractéristiques et pour la génération d'un champ électrique positif ou négatif entre la région de la tête et la région périphérique du patient pour former un signal de stimulation global modulaire avec la fréquence de commande sympathique ou parasympathique à basse fréquence à titre d'oscillations de base, ainsi que des fréquences électroencéphalographiques typiques à l'application et des oscillations sinusoïdales de plus hautes fréquences dans une série de Fourier d'une oscillation de base de 250 Hz ainsi que les oscillations d'ordre supérieur de 500, 750, 1000, 1250, 1500 Hz.

2. Dispositif selon la revendication 1,
    **caractérisé en ce que**, pour l'injection de l'énergie on utilise des applicateurs de champ qui garantissent l'établissement, au milieu du corps, dans la région supérieure du corps du patient, d'une concentration du flux magnétique qui est engendré par un applicateur de Matten, et, en prenant pour base les relations physiologiques dans la moitié inférieure du corps, un applicateur de Matten associé est ainsi réalisé que la répartition de l'énergie du champ se produit vers la région des deux jambes, donc les centres présentent une certaine distance, et qu'il en résulte ainsi deux maxima pour l'intensité du champ.

3. Dispositif selon la revendication 2,
    **caractérisé en ce que** les applicateurs de champ comprennent une combinaison d'un agencement à bobine qui engendre un champ magnétique et d'un agencement d'électrode qui engendre le champ électrique, afin de combiner l'avantage de la stimulation via les potentiels électriques avec l'apport des effets du champ magnétique, et dans lequel l'électrode qui engendre le champ électrique peut être simultanément conçue comme bobine qui engendre le champ magnétique.

4. Dispositif selon la revendication 3,
    **caractérisé en ce que** les applications ont lieu à partir de diverses bobines individuelles ou de bobine en combinaison avec des grilles ou des surfaces électroconductrices, qui permettent aussi bien l'alimentation de courant électrique que de tension électrique, et dans lequel par coopération des éléments des champs électriques et magnétiques sont amenés de façons combinée en action.

5. Dispositif selon l'une des revendications précédentes,
    **caractérisé en ce que**, au moyen d'un circuit oscillant bobine-condensateur accordé à la fréquence de résonance, on réalise les états d'affichage en coïncidence avec une fréquence caractéristique du signal de stimulation via un détecteur, afin de contrôler l'effet du dispositif de stimulation.

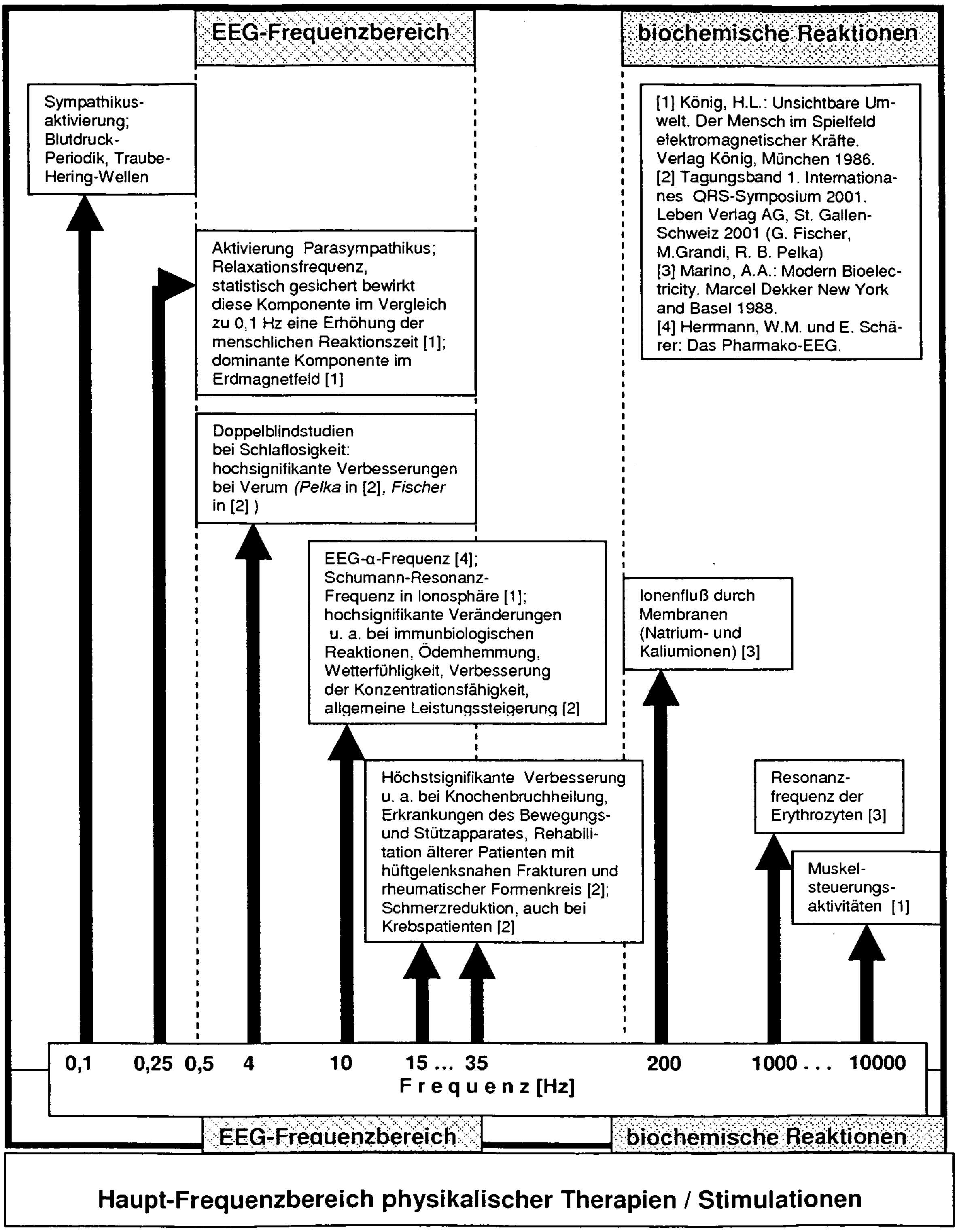
EEG-Frequenzbereich
biochemische Reaktionen
Sympathikus-aktivierung; Blutdruck-Periodik, Traube-Hering-Wellen
Aktivierung Parasympathikus; Relaxationsfrequenz, statistisch gesichert bewirkt diese Komponente im Vergleich zu 0,1 Hz eine Erhöhung der menschlichen Reaktionszeit [1]; dominante Komponente im Erdmagnetfeld [1]
Doppelblindstudien bei Schlaflosigkeit: hochsignifikante Verbesserungen bei Verum (Pelka in [2], Fischer in [2] )
EEG-α-Frequenz [4]; Schumann-Resonanz-Frequenz in Ionosphäre [1]; hochsignifikante Veränderungen u. a. bei immunbiologischen Reaktionen, Ödemhemmung, Wetterfühligkeit, Verbesserung der Konzentrationsfähigkeit, allgemeine Leistungssteigerung [2]
Höchstsignifikante Verbesserung u. a. bei Knochenbruchheilung, Erkrankungen des Bewegungs- und Stützapparates, Rehabilitation älterer Patienten mit hüftgelenksnahen Frakturen und rheumatischer Formenkreis [2]; Schmerzreduktion, auch bei Krebspatienten [2]
[1] König, H.L.: Unsichtbare Umwelt. Der Mensch im Spielfeld elektromagnetischer Kräfte. Verlag König, München 1986.
[2] Tagungsband 1. Internationanes QRS-Symposium 2001. Leben Verlag AG, St. Gallen-Schweiz 2001 (G. Fischer, M.Grandi, R. B. Pelka)
[3] Marino, A.A.: Modern Bioelectricity. Marcel Dekker New York and Basel 1988.
[4] Herrmann, W.M. und E. Schärer: Das Pharmako-EEG.
Ionenfluß durch Membranen (Natrium- und Kaliumionen) [3]
Resonanzfrequenz der Erythrozyten [3]
Muskelsteuerungsaktivitäten [1]
0,1 0,25 0,5 4 10 15 ... 35 200 1000 ... 10000
Frequenz [Hz]
EEG-Frequenzbereich
biochemische Reaktionen
Haupt-Frequenzbereich physikalischer Therapien / Stimulationen

Fig. 1

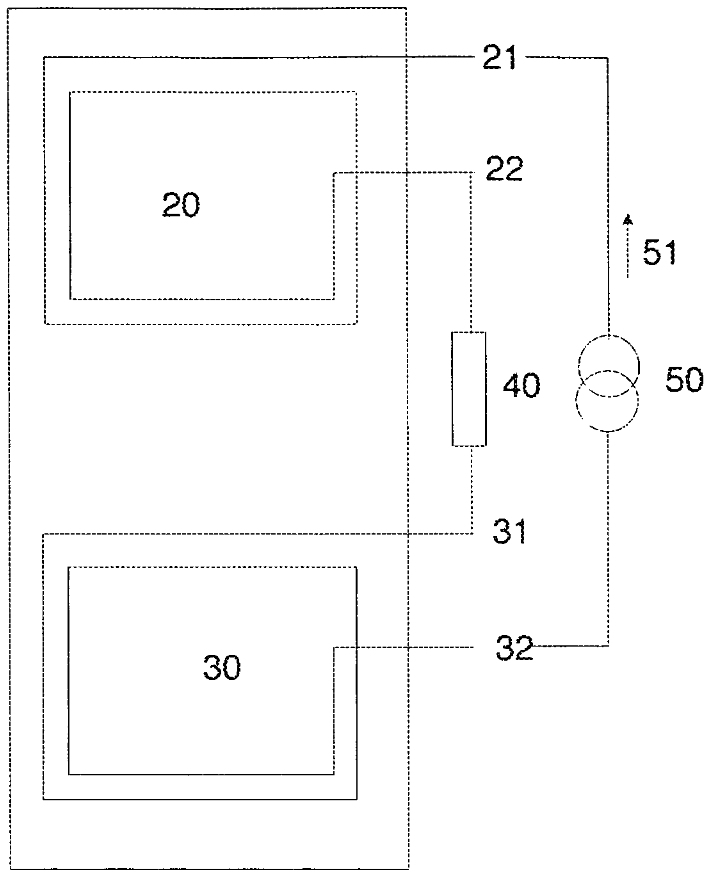


Fig. 2

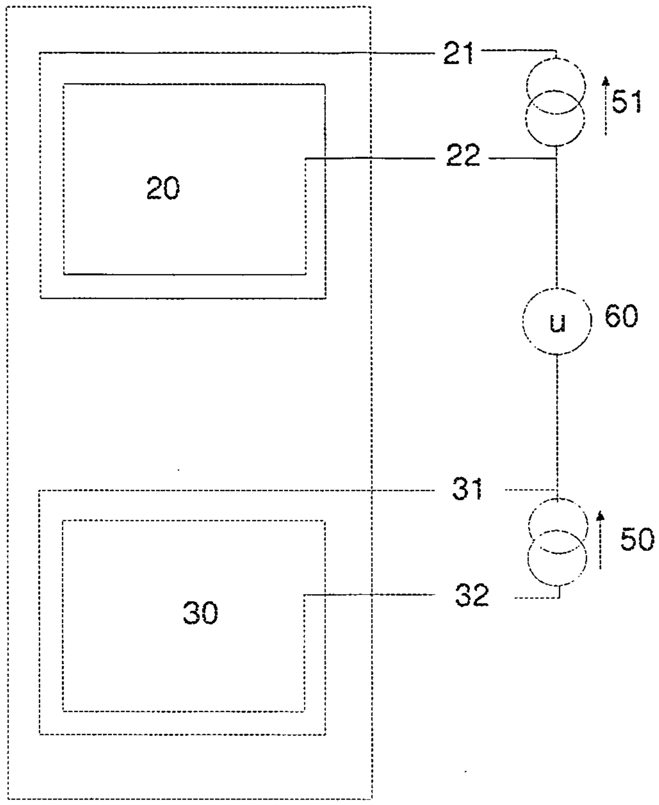


Fig. 3

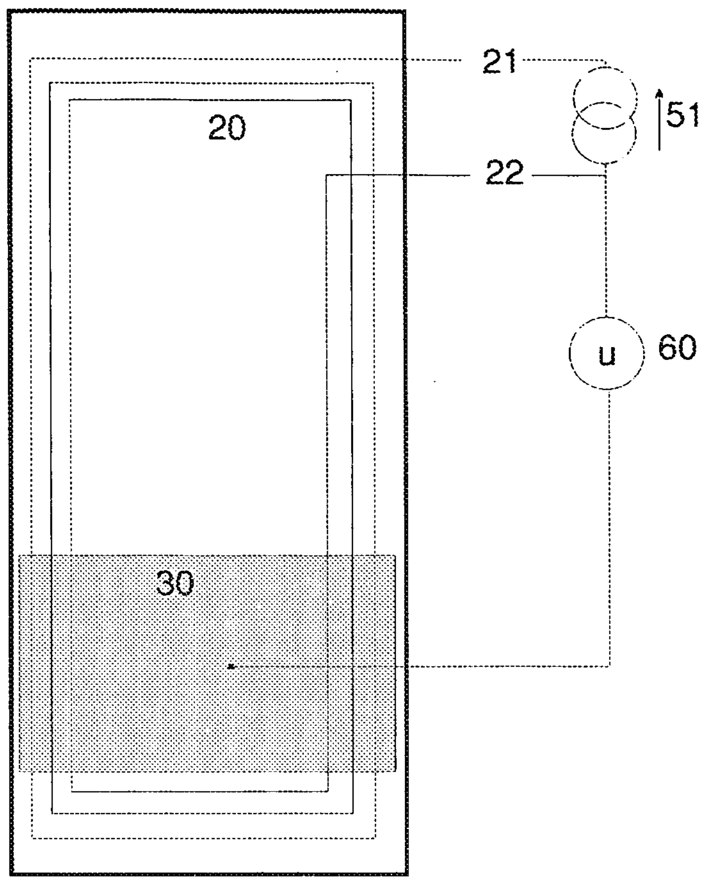


Fig. 4

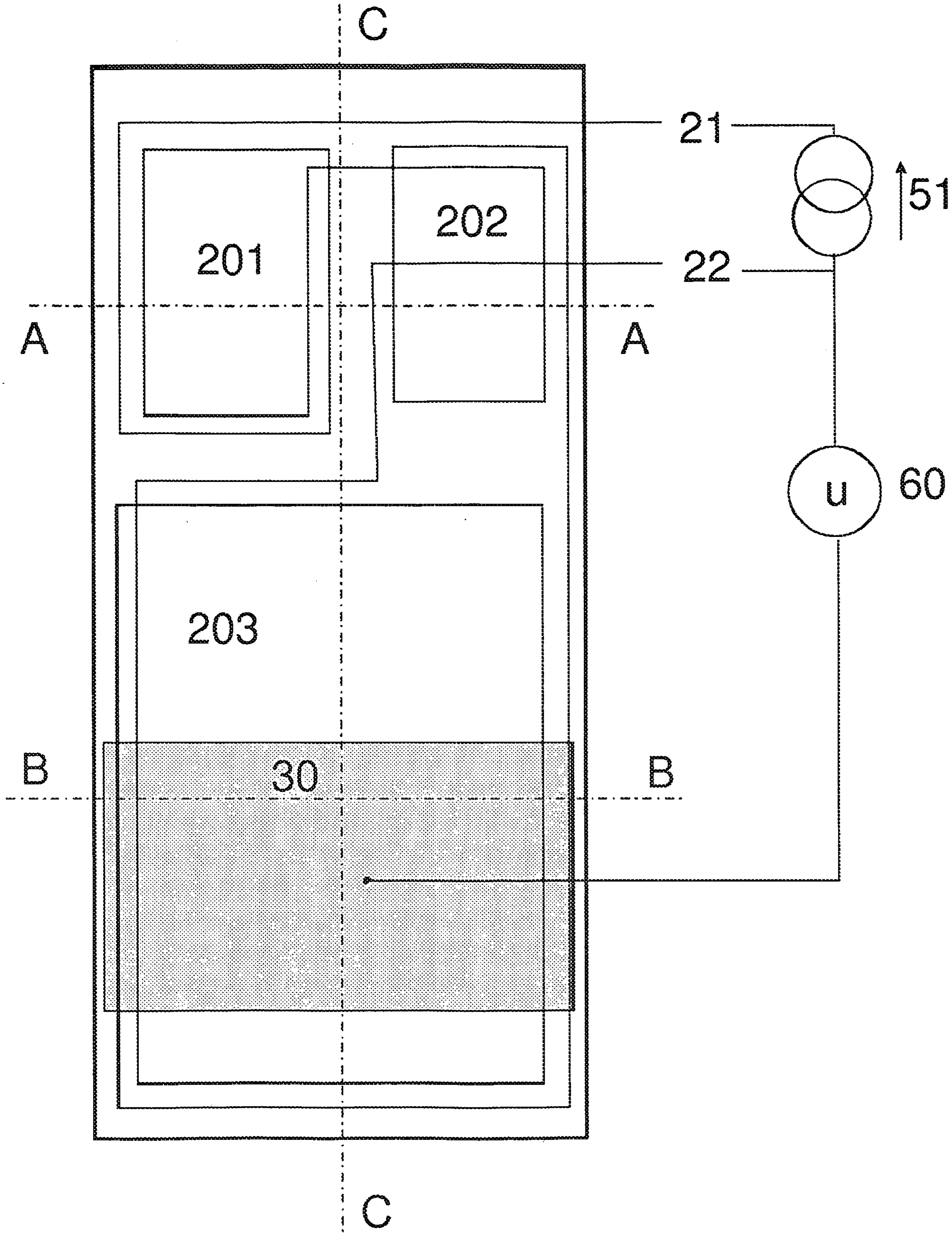


Fig. 5

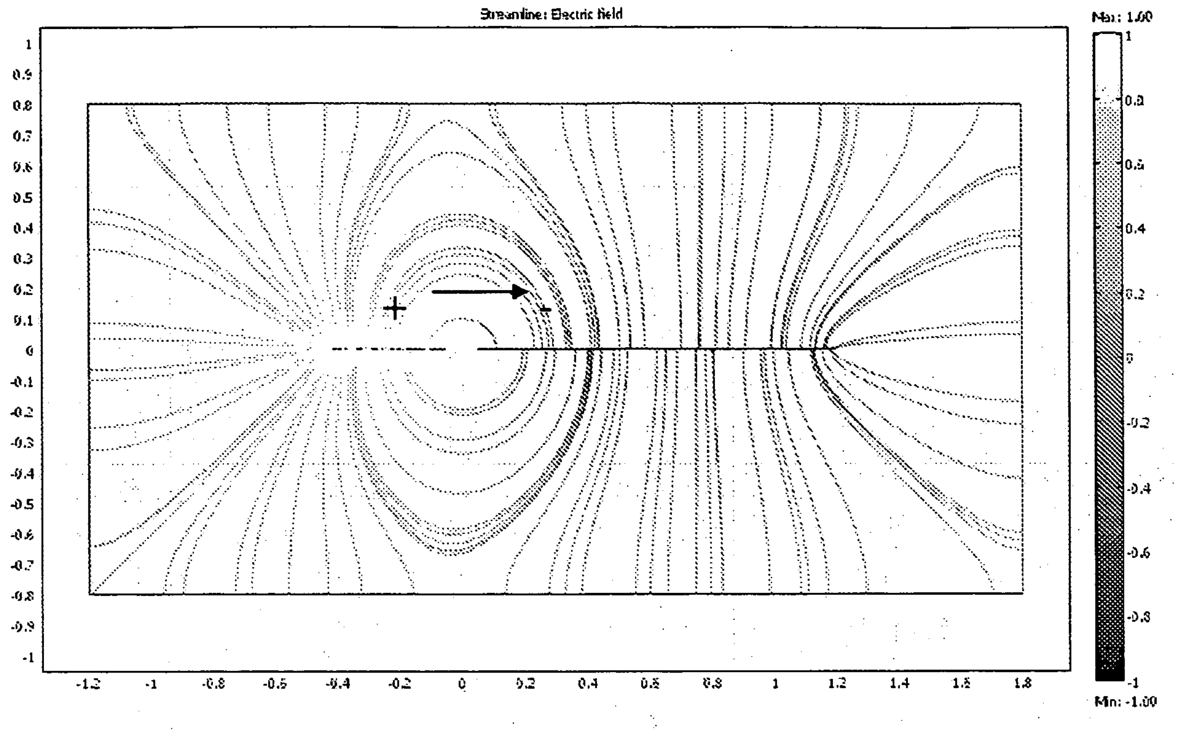


Fig. 6

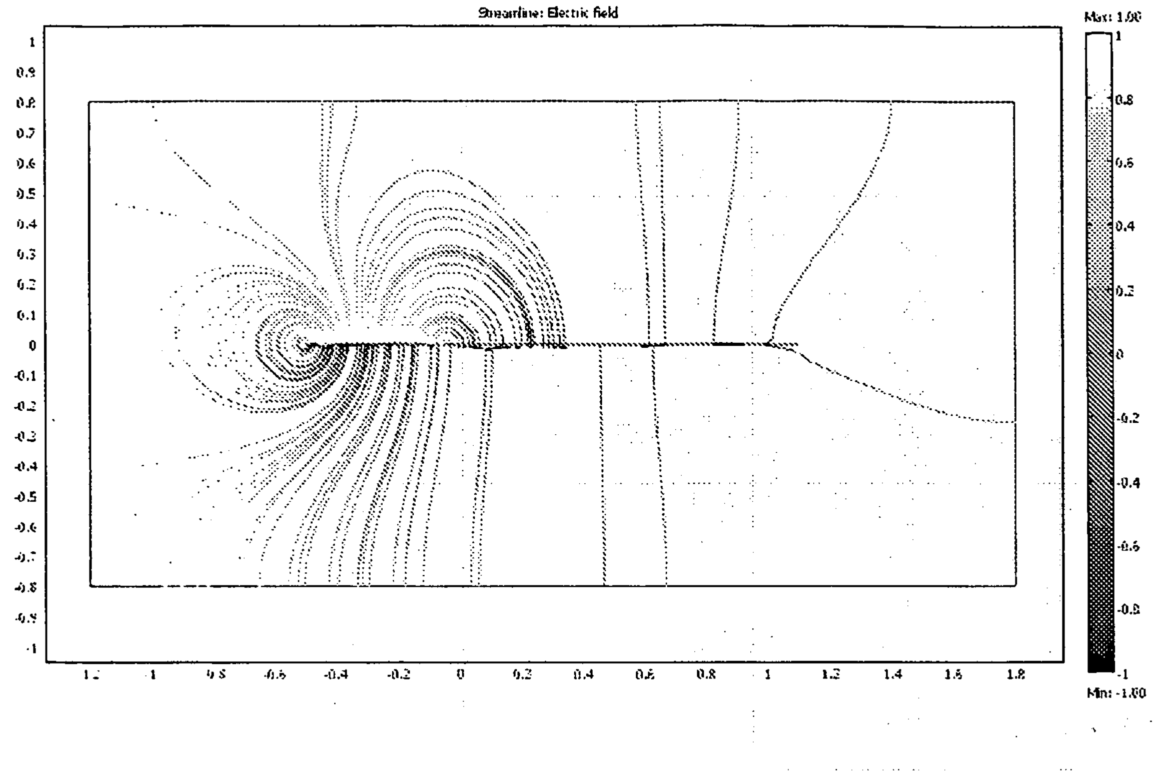


Fig. 7

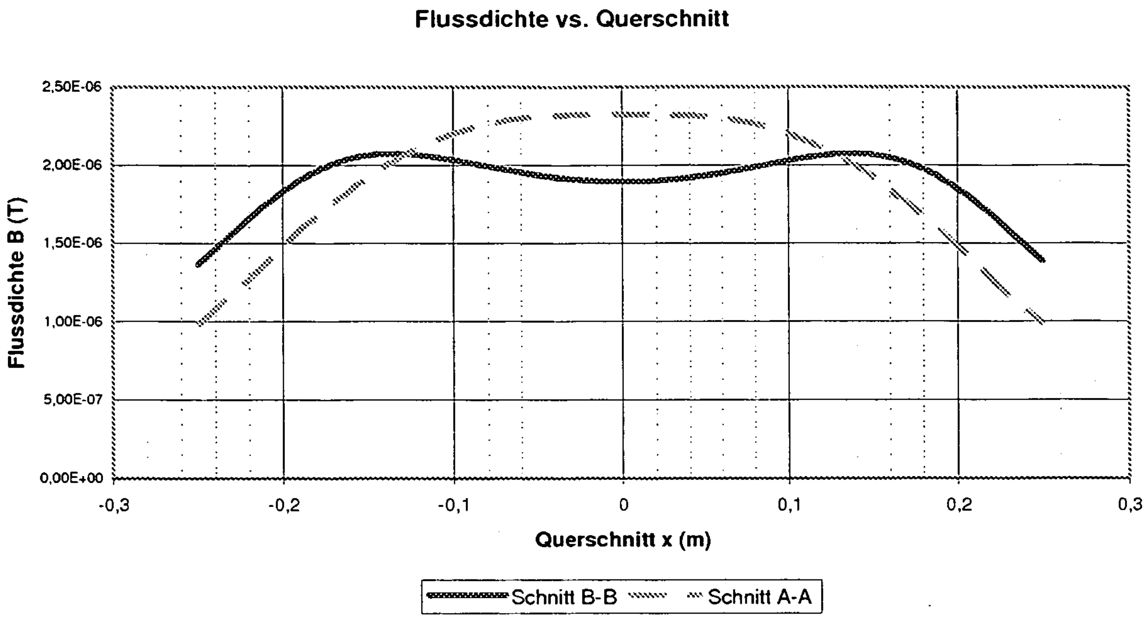
Flussdichte vs. Querschnitt
Flussdichte B (T)
2,50E-06
2,00E-06
1,50E-06
1,00E-06
5,00E-07
0,00E+00
-0,3
-0,2
-0,1
0
0,1
0,2
0,3
Querschnitt x (m)
Schnitt B-B
Schnitt A-A

Fig. 8

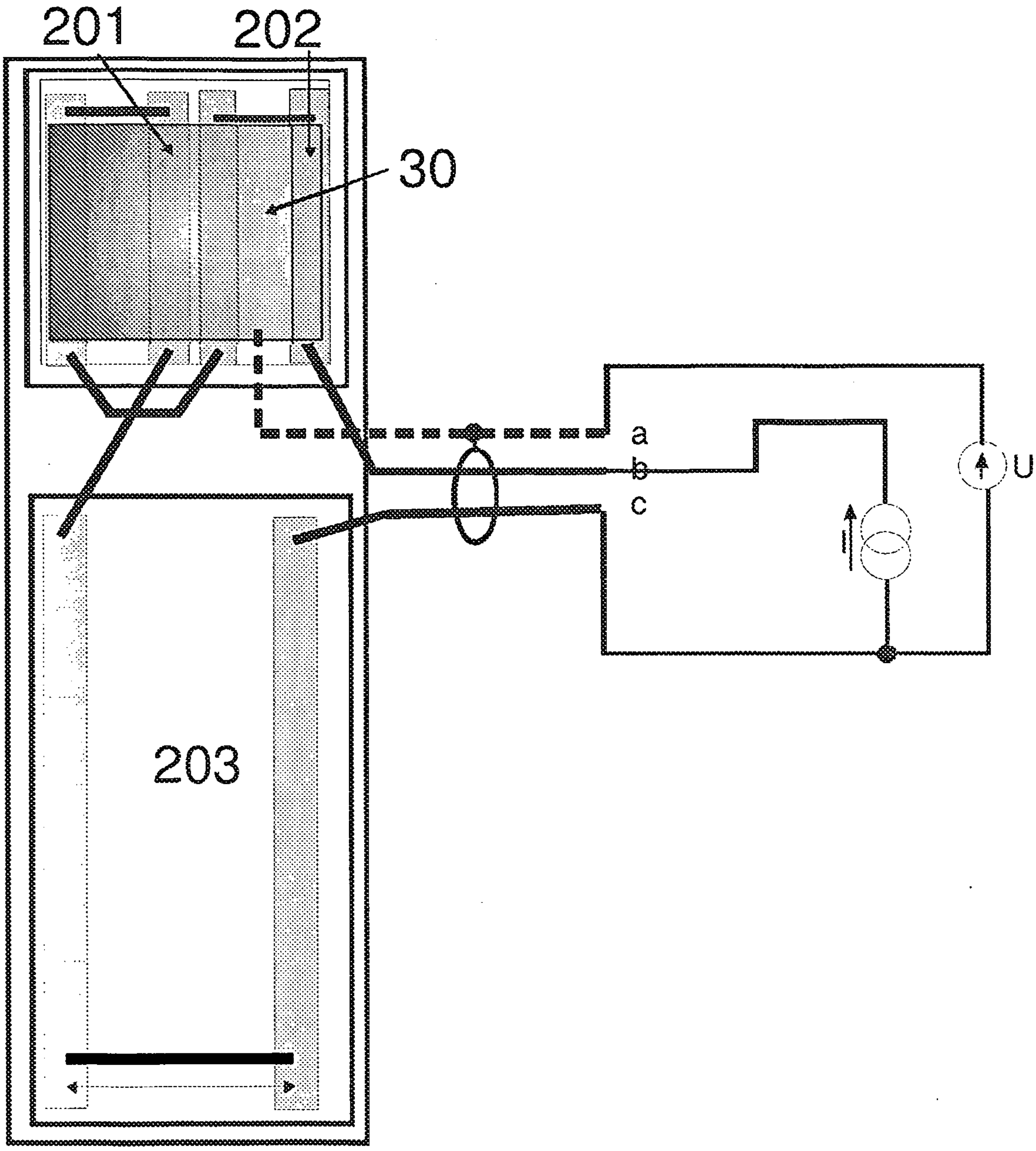


Fig. 9

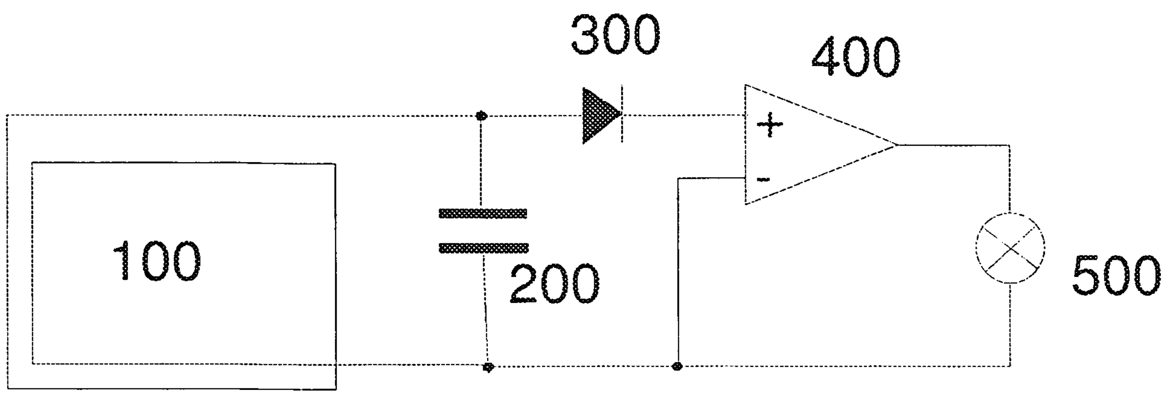


Figur 10

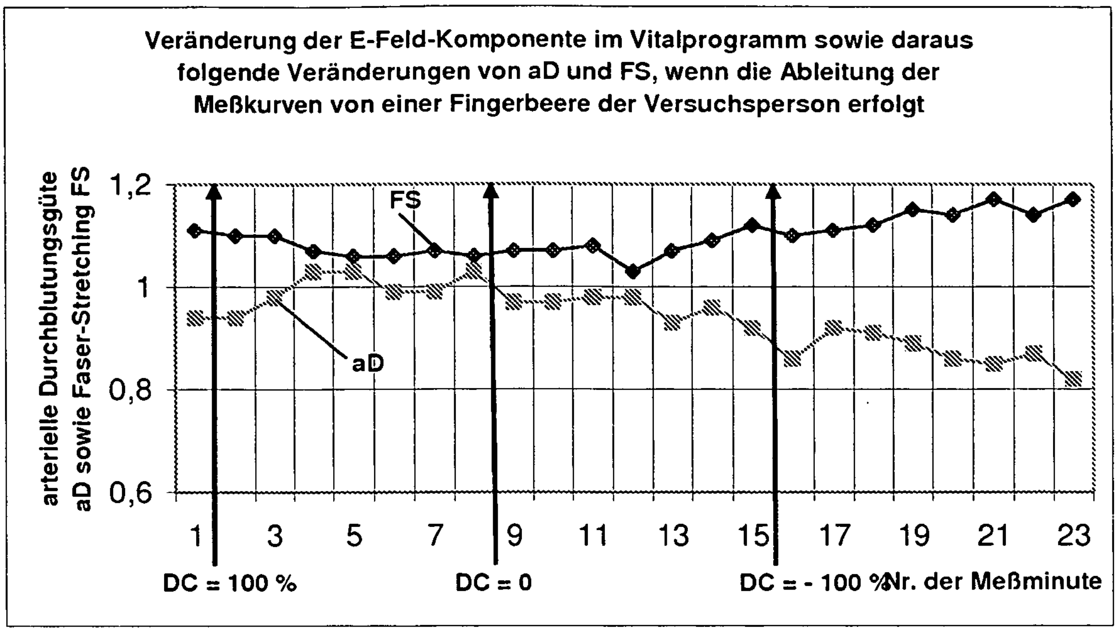
Veränderung der E-Feld-Komponente im Vitalprogramm sowie daraus folgende Veränderungen von aD und FS, wenn die Ableitung der Meßkurven von einer Fingerbeere der Versuchsperson erfolgt
arterielle Durchblutungsgüte aD sowie Faser-Stretching FS
1,2
1
0,8
0,6
FS
aD
1
3
5
7
9
11
13
15
17
19
21
23
DC = 100 %
DC = 0
DC = - 100 %
Nr. der Meßminute

Fig. 11

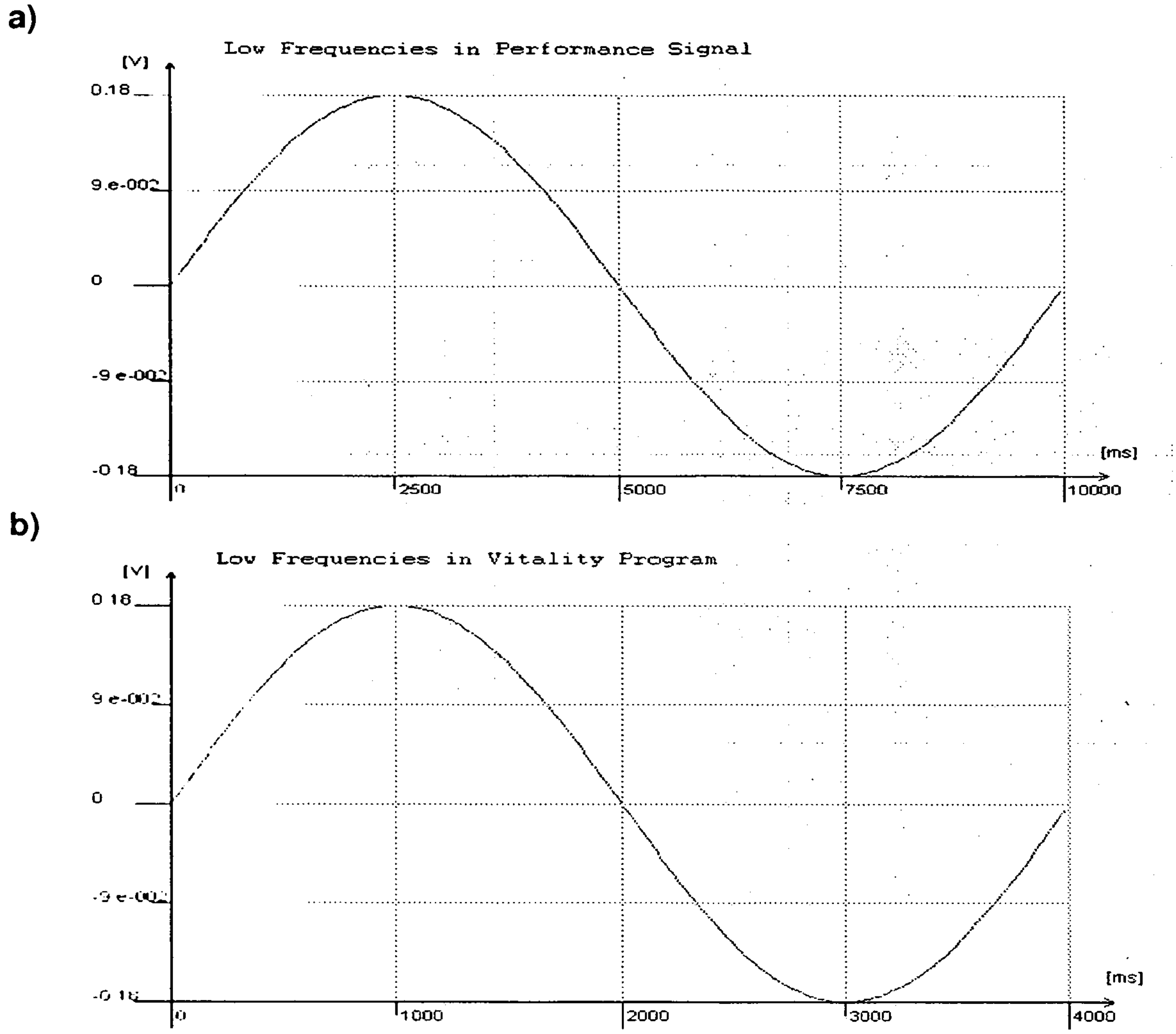
a)
Low Frequencies in Performance Signal
[V]
0.18
9.e-002
0
-9 e-002
-0.18
0
2500
5000
7500
10000
[ms]
b)
Low Frequencies in Vitality Program
[V]
0.18
9 e-002
0
-9 e-002
-0.18
0
1000
2000
3000
4000
[ms]

Fig. 12

| | Frequenz [Hz] | Faktor [%] | Phase [°] |
|---|---|---|---|
| Signal 1 | 250 | 60 | 180 |
| Signal 2 | 500 | 30 | 180 |
| Signal 3 | 750 | 20 | 180 |
| Signal 4 | 1000 | 15 | 180 |
| Signal 5 | 1250 | 12 | 180 |
| Signal 6 | 0 | 0 | 0 |
| Signal 7 | 0 | 0 | 0 |
| Signal 8 | 0 | 0 | 0 |
| Signal 9 | 0 | 0 | 0 |
| Signal 10 | 0 | 0 | 0 |
| Signal 11 | 0 | 0 | 0 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

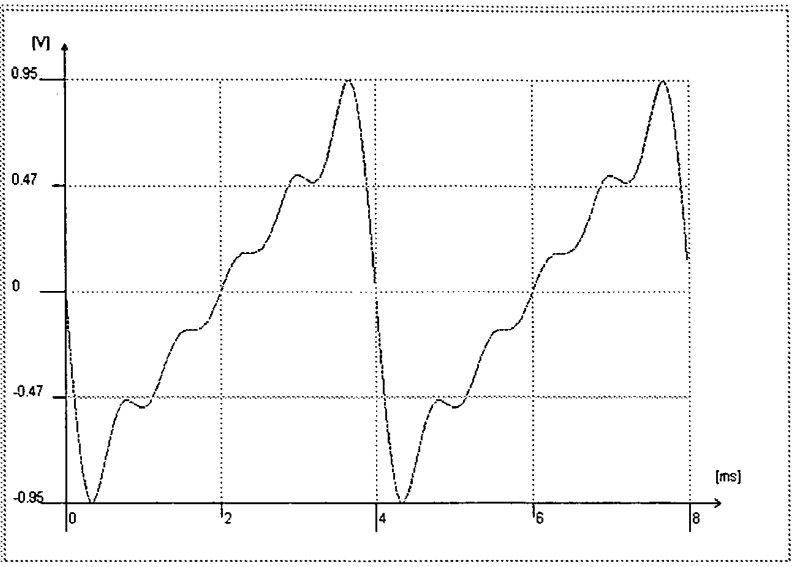


Fig. 13

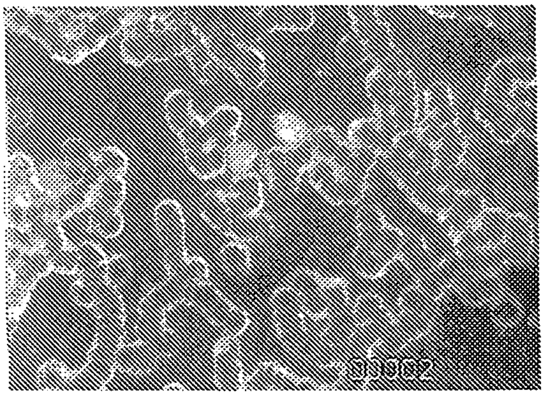
vor Stimulation

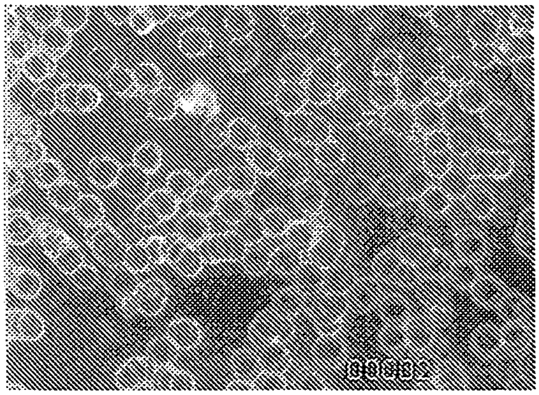
nach 2 min IIS-Stimulation

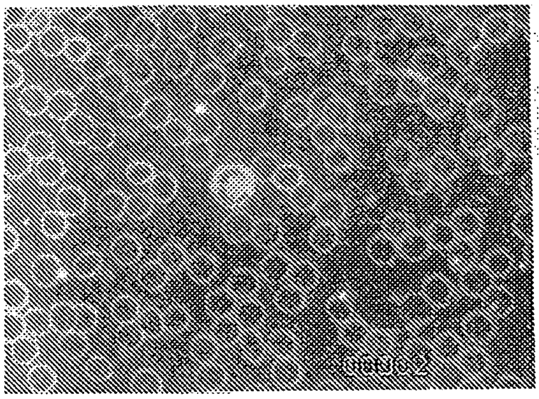
nach 4 min IIS-Stimulation

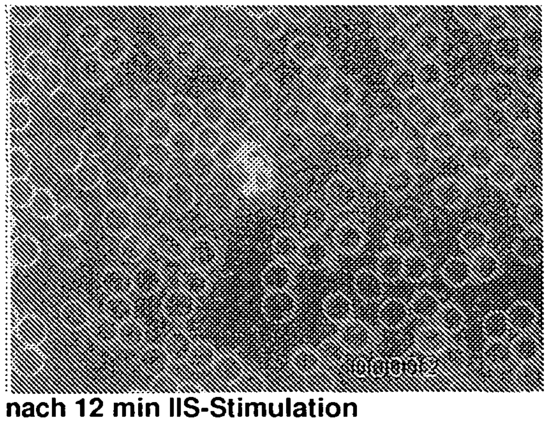
nach 12 min IIS-Stimulation

Fig.14

a)

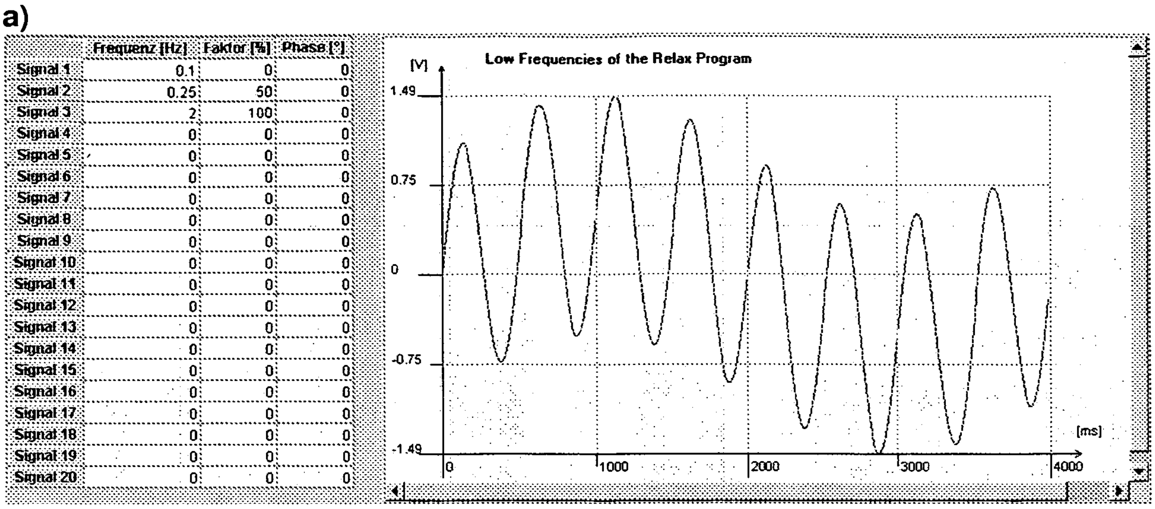

| | Frequenz [Hz] | Faktor [%] | Phase [°] |
|---|---|---|---|
| Signal 1 | 0.1 | 0 | 0 |
| Signal 2 | 0.25 | 50 | 0 |
| Signal 3 | 2 | 100 | 0 |
| Signal 4 | 0 | 0 | 0 |
| Signal 5 | 0 | 0 | 0 |
| Signal 6 | 0 | 0 | 0 |
| Signal 7 | 0 | 0 | 0 |
| Signal 8 | 0 | 0 | 0 |
| Signal 9 | 0 | 0 | 0 |
| Signal 10 | 0 | 0 | 0 |
| Signal 11 | 0 | 0 | 0 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

Low Frequencies of the Relax Program
[V]
1.49
0.75
0
-0.75
-1.49
0
1000
2000
3000
4000
[ms]


b)

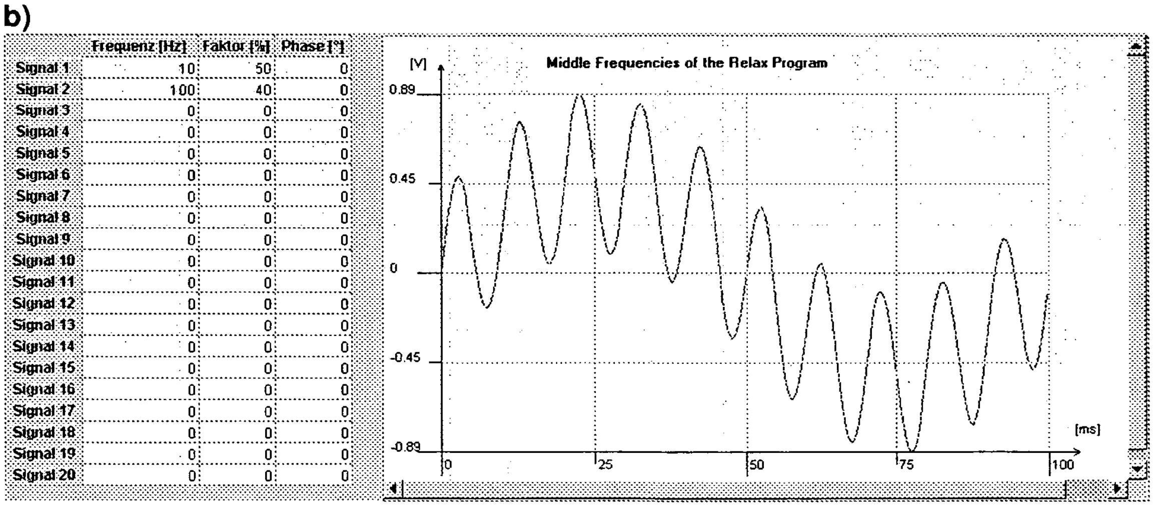

| | Frequenz [Hz] | Faktor [%] | Phase [°] |
|---|---|---|---|
| Signal 1 | 10 | 50 | 0 |
| Signal 2 | 100 | 40 | 0 |
| Signal 3 | 0 | 0 | 0 |
| Signal 4 | 0 | 0 | 0 |
| Signal 5 | 0 | 0 | 0 |
| Signal 6 | 0 | 0 | 0 |
| Signal 7 | 0 | 0 | 0 |
| Signal 8 | 0 | 0 | 0 |
| Signal 9 | 0 | 0 | 0 |
| Signal 10 | 0 | 0 | 0 |
| Signal 11 | 0 | 0 | 0 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

Middle Frequencies of the Relax Program
[V]
0.89
0.45
0
-0.45
-0.89
0
25
50
75
100
[ms]


c)

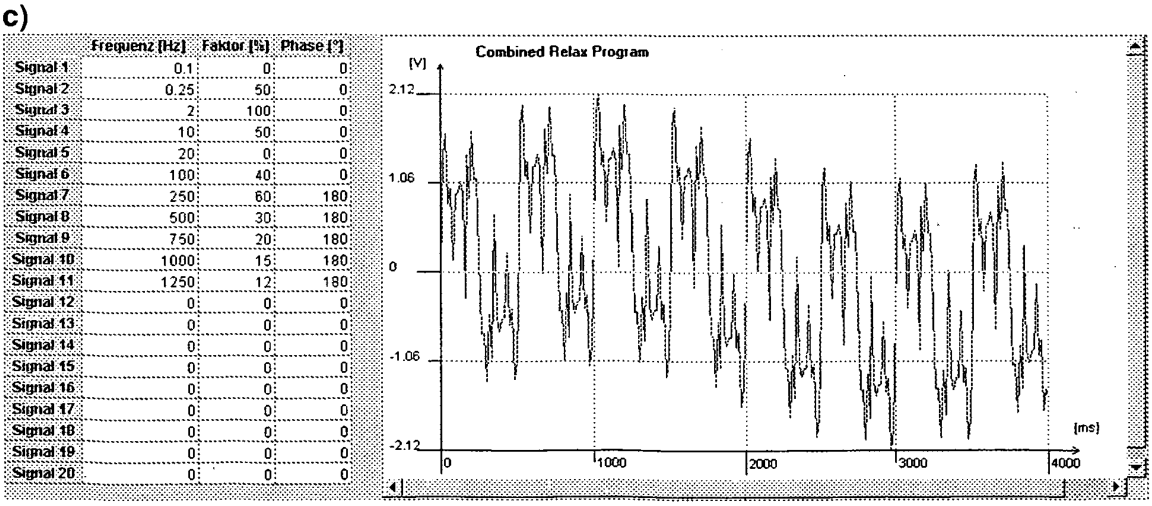

| | Frequenz [Hz] | Faktor [%] | Phase [°] |
|---|---|---|---|
| Signal 1 | 0.1 | 0 | 0 |
| Signal 2 | 0.25 | 50 | 0 |
| Signal 3 | 2 | 100 | 0 |
| Signal 4 | 10 | 50 | 0 |
| Signal 5 | 20 | 0 | 0 |
| Signal 6 | 100 | 40 | 0 |
| Signal 7 | 250 | 60 | 180 |
| Signal 8 | 500 | 30 | 180 |
| Signal 9 | 750 | 20 | 180 |
| Signal 10 | 1000 | 15 | 180 |
| Signal 11 | 1250 | 12 | 180 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

Combined Relax Program
[V]
2.12
1.06
0
-1.06
-2.12
0
1000
2000
3000
4000
[ms]


Fig.15

**a)**

| | Frequenz [Hz] | Faktor [%] | Phase [°] |
|---|---|---|---|
| Signal 1 | 10 | 100 | 0 |
| Signal 2 | 20 | 0 | 0 |
| Signal 3 | 100 | 40 | 0 |
| Signal 4 | 0 | 0 | 0 |
| Signal 5 | 0 | 0 | 0 |
| Signal 6 | 0 | 0 | 0 |
| Signal 7 | 0 | 0 | 0 |
| Signal 8 | 0 | 0 | 0 |
| Signal 9 | 0 | 0 | 0 |
| Signal 10 | 0 | 0 | 0 |
| Signal 11 | 0 | 0 | 0 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

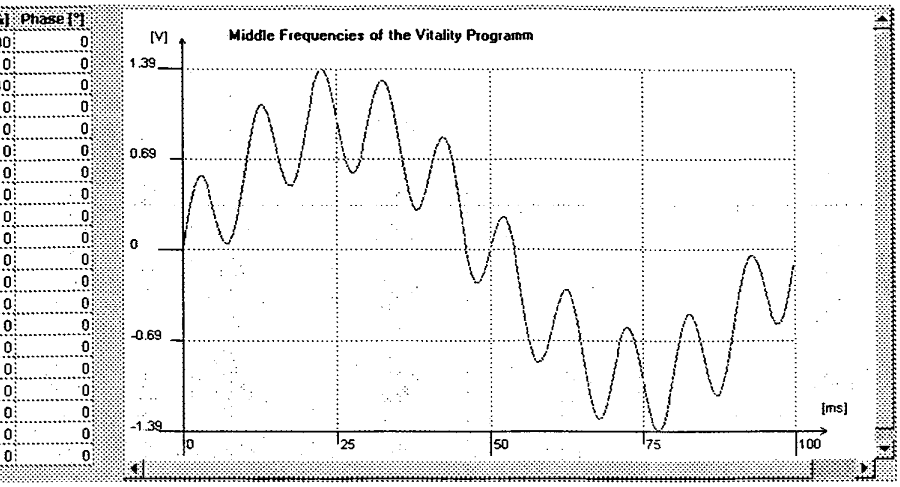


**b)**

| | Frequenz [Hz] | Faktor [%] | Phase [°] |
|---|---|---|---|
| Signal 1 | 0.1 | 0 | 0 |
| Signal 2 | 0.25 | 50 | 0 |
| Signal 3 | 2 | 0 | 0 |
| Signal 4 | 10 | 100 | 0 |
| Signal 5 | 20 | 0 | 0 |
| Signal 6 | 100 | 40 | 0 |
| Signal 7 | 250 | 60 | 180 |
| Signal 8 | 500 | 30 | 180 |
| Signal 9 | 750 | 20 | 180 |
| Signal 10 | 1000 | 15 | 180 |
| Signal 11 | 1250 | 12 | 180 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

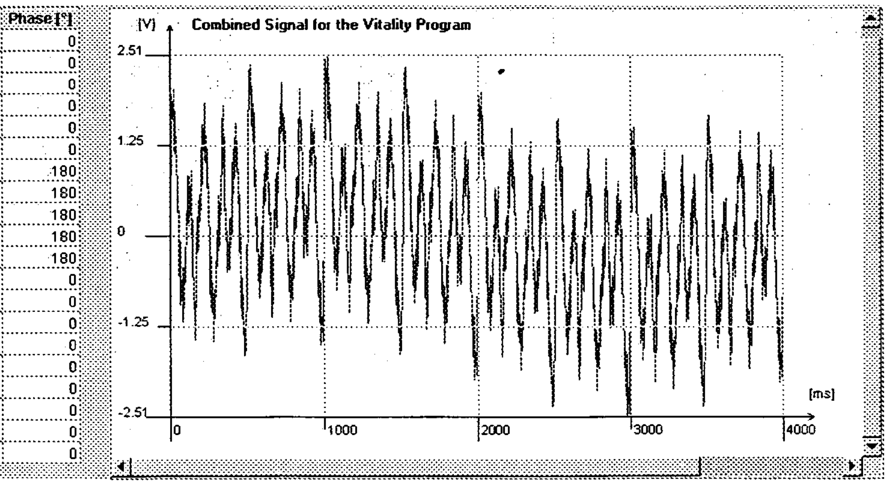


Fig. 16

**a)**

| | Frequenz [Hz] | Faktor [%] | Phase [°] |
|---|---|---|---|
| Signal 1 | 10 | 100 | 0 |
| Signal 2 | 20 | 70 | 0 |
| Signal 3 | 100 | 40 | 0 |
| Signal 4 | 0 | 0 | 0 |
| Signal 5 | 0 | 0 | 0 |
| Signal 6 | 0 | 0 | 0 |
| Signal 7 | 0 | 0 | 0 |
| Signal 8 | 0 | 0 | 0 |
| Signal 9 | 0 | 0 | 0 |
| Signal 10 | 0 | 0 | 0 |
| Signal 11 | 0 | 0 | 0 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

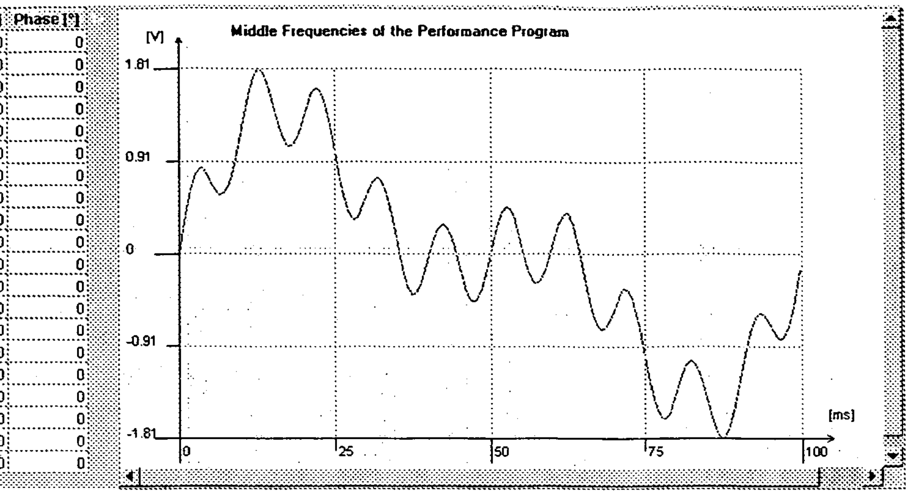


**b)**

| | Frequenz [Hz] | Faktor [%] | Phase [°] |
|---|---|---|---|
| Signal 1 | 0.1 | 30 | 0 |
| Signal 2 | 0.25 | 0 | 0 |
| Signal 3 | 2 | 0 | 0 |
| Signal 4 | 10 | 100 | 0 |
| Signal 5 | 20 | 70 | 0 |
| Signal 6 | 100 | 40 | 0 |
| Signal 7 | 250 | 60 | 180 |
| Signal 8 | 500 | 30 | 180 |
| Signal 9 | 750 | 20 | 180 |
| Signal 10 | 1000 | 15 | 180 |
| Signal 11 | 1250 | 12 | 180 |
| Signal 12 | 0 | 0 | 0 |
| Signal 13 | 0 | 0 | 0 |
| Signal 14 | 0 | 0 | 0 |
| Signal 15 | 0 | 0 | 0 |
| Signal 16 | 0 | 0 | 0 |
| Signal 17 | 0 | 0 | 0 |
| Signal 18 | 0 | 0 | 0 |
| Signal 19 | 0 | 0 | 0 |
| Signal 20 | 0 | 0 | 0 |

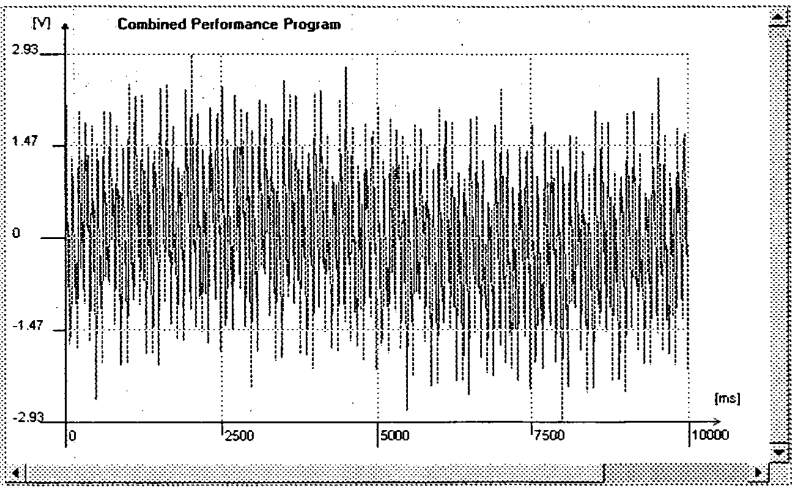


Fig. 17

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6675047 B1


### In der Beschreibung aufgeführte Nicht-Patentliteratur


- Unsichtbare Umwelt. *Der Mensch im Spielfeld elektromagnetischer Kräfte,* 1986
- Modern Bioelectricity. Marcel Dekker, 1988
- Modern Problems of Pharmacopsychiatry. Psychotropic Drugs and the Human EEG. 1974, vol. 8
- CEAN-computerized EEG-Analysis. Fischer, 1975
- **Herrmann, W. M. ; E. Schärer.** *Das Pharmako-EEG. Grundlagen, Methodik, Anwendung. Landsberg / Lech / Germany,* 1987, ISBN 3-609-64170-3
- **Fournier.** Description des installations dúne station dénregistrement des variations tre’s rapides du champ magne’tique terrestre. *extrait des Comptes Rendus des seánces de l ’Acade’mie des Sciences,* 01. August 1960, vol. 251, 671-673
- **König.** Unsichtbare Umwelt. Der Mensch im Spielfeld elektromagnetischer Kräfte. *Eigenverlag Herbert L König,* 1986
- Possible Cardiac Driving by an External Rotating magnetic Field. *Int. J. Biometeor.,* 1973, vol. 17 (3), 263-266
- **Thews, G ; R Schmidt.** Human Physiologie. Springer, 1989
- **Friedmann, H. ; R. O. Becker ; C. H. Bachmann.** Effect of Magnetic Fields on Reaction Time Performance. *Nature,* 1967, vol. 213 (5079), 949-950
- **Krauß, M.** Die natürlichen elektromagnetischen Signale in unserer Umwelt und deren Simulation als QRS®-Magnetfeldtherapie. *Vortrag auf dem Norddeutschen Kongress für komplementäre Medizin,* 22. Juni 2002